# EUROPÄISCHE PATENTSCHRIFT

(11) **EP 2 663 626 B1**
(45) Veröffentlichungstag und Bekanntmachung des Hinweises auf die Patenterteilung: **08.05.2019**
(21) Anmeldenummer: 12700184.0
(22) Anmeldetag: 12.01.2012
(51) Int. Cl.: C08L 71/02, C11D 1/755, C11D 1/00, C08G 65/334

(54) **VERWENDUNG VON GEGEBENENFALLS OXIDIERTEN THIOETHERN VON POLYALKYLENOXIDEN IN WASCH- UND REINIGUNGSMITTELN**
USE OF OPTIONALLY OXIDIZED THIOETHERS OF POLYALKYLENE GLYCOLS IN DETERGENTS AND CLEANING AGENTS
UTILISATION DE THIOÉTHERS ÉVENTUELLEMENT OXYDÉS DE POLY(OXYDES D'ALKYLÈNE) DANS DES AGENTS DE LAVAGE ET DE NETTOYAGE

(30) Priorität: 13.01.2011 EP 11150876
(43) Veröffentlichungstag der Anmeldung: 20.11.2013
(73) Patentinhaber: BASF SE, 67056 Ludwigshafen am Rhein (DE)
(72) Erfinder: MAITRO-VOGEL, Sophie, 68199 Mannheim (DE); TROPSCH, Jürgen, 67354 Römerberg (DE); SPIEGLER, Wolfgang, 67551 Worms (DE); RAETHER, Roman, Benedikt, 67346 Speyer (DE)
(86) Internationale Anmeldenummer: PCT/EP2012/050422
(87) Internationale Veröffentlichungsnummer: WO 2012/095482

(56) Entgegenhaltungen:
- WO-A1-93/02387
- GB-A- 643 456
- US-A- 3 288 858
- US-A- 4 663 082

## Beschreibung

Die vorliegende Erfindung betrifft die Verwendung von (oxidierten) Thioethern von Polyalkylenoxiden in Wasch- und Reinigungsmitteln, insbesondere in Geschirrspülmitteln, und Wasch- und Reinigungsmittel, insbesondere Geschirrspülmittel, die (oxidierte) Thioether von Alkoholalkoxylaten enthalten. Diese (oxidierten) Thioether sind dabei insbesondere als Tenside mit Klarspülfunktion (Klarspültenside) geeignet. "Oxidiert" bezieht sich auf das Schwefelatom im Thioether, das in oxidierter Form als Sulfoxid (SO) oder Sulfonyl (SO₂) vorliegen kann. Außerdem betrifft die Erfindung bestimmte oxidierte Thioether von Polyalkylenoxiden.

Tenside sind Substanzen, die die Grenzflächenspannung herabsetzen können. Typischerweise verfügen Tenside über einen charakteristischen Aufbau und weisen mindestens eine hydrophile und mindestens eine hydrophobe funktionelle Gruppe auf. Wenn beide Teile des Moleküls im Gleichgewicht zueinander stehen, wird sich die Substanz an einer Grenzfläche anreichern und ausrichten, d.h. hydrophile Gruppen weisen beispielsweise in eine wässrige Phase und die hydrophoben Gruppen in Richtung anderer fester, flüssiger oder gasförmiger Phasen. Eine weitere Besonderheit von Tensiden ist die Bildung höherer Aggregate, den sogenannten Micellen. Bei diesen ordnen sich die Tensidmoleküle dergestalt an, dass die polaren Gruppen zum Beispiel eine Kugelschale bilden. Dies bewirkt, dass Stoffe wie Schmutzpartikel in einer wässrigen Lösung unter Ausbildung von Micellen löslich gemacht werden. Daher eignen sich Tenside insbesondere zur Reinigung von Oberflächen und als Zusatz in Waschmitteln.

Tenside, die einen hydrophoben und einen hydrophilen Block aufweisen, sind weit verbreitet. Ihre Neigung zur Schaumbildung macht sie aber für viele Anwendungen nicht oder nur bedingt brauchbar. Für Anwendungen, in denen eine starke Schaumbildung nicht erwünscht ist, wurden daher nichtionische Tenside entwickelt, die einen zweiten hydrophoben Block aufweisen, was das Schaumvolumen begrenzt.

Der zweite hydrophobe Block kann sich beispielsweise von einem Fettalkohol ableiten. Allerdings führt die Verwendung von Geschirrspülmitteln, die ein solches Tensid enthalten, insbesondere von Geschirrspülmitteln für Spülmaschinen, häufig dazu, dass auf dem damit gereinigten Geschirr Rückstände zurückbleiben (Belagsbildung; sogenanntes "spotting" im Falle der Bildung von punktuellen Belägen bzw. "filming" im Falle von filmartigen Belägen).

Der zweite hydrophobe Block kann sich alternativ von einer Fettsäure ableiten. Bei Geschirrspülmitteln, die solche Tenside enthalten, ist zwar das Problem der Belagsbildung nicht mehr so groß; allerdings sind diese Tenside aufgrund der Estergruppe hydrolyseempfindlich, was ihre Verwendbarkeit in alkalischen Formulierungen und bei höheren Temperaturen, insbesondere bei längeren Waschvorgängen, stark einschränkt.

Thioether von Polyalkylenoxiden sind bekannt; beispielsweise aus US 5,593,953, wo ihre Verwendung als Schmierstoffadditive beschrieben wird, oder aus der WO 93/02387, wo sie in photographischen Hochkontrastelementen zur Verhinderung des Pfefferschleiers (pepper fog) eingesetzt werden. Eine Verwendung als Wasch- oder Reinigungsmittel ist aus US4663082, US3288858 und GB643456 erwähnt.

Aufgabe der vorliegenden Erfindung war es, Verbindungen bereitzustellen, die die Nachteile der Tenside des Standes der Technik nicht aufweisen. Insbesondere sollten die Verbindungen keine bzw. keine starke Neigung zur Schaumbildung haben; sie sollten weniger Rückstände, insbesondere filming-Rückstände, auf damit gewaschenem Geschirr hinterlassen, und sie sollten nicht oder zumindest weniger hydrolyselabil sein als die Tenside auf Fettsäureester-Basis.

Die Aufgabe wird gelöst durch die Verwendung von Verbindungen der Formel I

R-S(O)ₓ-[-CH₂CH₂-O-]ₙ-CH₂CH₂-S(O)_{y}-R' (I)

worin
R und R' unabhängig voneinander für C₆-C₁₈-Alkyl stehen;
n für eine Zahl von 11 bis 150 steht; und
x und y unabhängig voneinander für 0, 1 oder 2 stehen;

in Wasch- oder Reinigungsmitteln, insbesondere in Geschirrspülmitteln. Außerdem betrifft die Erfindung Wasch- oder Reinigungsmittel, insbesondere Geschirrspülmittel, enthaltend wenigstens eine Verbindung der Formel I gemäß obiger Definition.

Im Rahmen der vorliegenden Erfindung steht C₆-C₁₈-Alkyl für einen linearen oder verzweigten Alkylrest mit 6 bis 18 Kohlenstoffatomen. Beispiele hierfür sind Hexyl, Heptyl, Octyl, 2-Ethylhexyl, Nonyl, Decyl, 2-Propylheptyl, Undecyl, Dodecyl, Tridecyl, Tetradecyl, Pentadecyl, Hexadecyl, Heptadecyl, Octadecyl und Stellungsisomere davon.

C₈-C₁₆-Alkyl für einen linearen oder verzweigten Alkylrest mit 8 bis 16 Kohlenstoffatomen. Beispiele hierfür sind Octyl, 2-Ethylhexyl, Nonyl, Decyl, 2-Propylheptyl, Undecyl, Dodecyl, Tridecyl, Tetradecyl, Pentadecyl, Hexadecyl und Stellungsisomere davon.

C₈-C₁₈-Alkyl steht für einen linearen oder verzweigten Alkylrest mit 8 bis 18 Kohlenstoffatomen. Beispiele hierfür sind Octyl, 2-Ethylhexyl, Nonyl, Decyl, 2-Propylheptyl, Undecyl, Dodecyl, Tridecyl, Tetradecyl, Pentadecyl, Hexadecyl, Heptadecyl, Octadecyl und Stellungsisomere davon.

C₈-C₁₄-Alkyl für einen linearen oder verzweigten Alkylrest mit 8 bis 14 Kohlenstoffatomen. Beispiele hierfür sind Octyl, 2-Ethylhexyl, Nonyl, Decyl, 2-Propylheptyl, Undecyl, Dodecyl, Tridecyl, Tetradecyl und Stellungsisomere davon.

C₁₀-C₁₈-Alkyl steht für einen linearen oder verzweigten Alkylrest mit 10 bis 18 Kohlenstoffatomen. Beispiele hierfür sind Decyl, 2-Propylheptyl, Undecyl, Dodecyl, Tridecyl, Tetradecyl, Pentadecyl, Hexadecyl, Heptadecyl, Octadecyl und Stellungsisomere davon.

C₁₀-C₁₄-Alkyl steht für einen linearen oder verzweigten Alkylrest mit 10 bis 14 Kohlenstoffatomen. Beispiele hierfür sind Decyl, 2-Propylheptyl, Undecyl, Dodecyl, Tridecyl, Tetradecyl und Stellungsisomere davon.

C₁₂-C₁₈-Alkyl steht für einen linearen oder verzweigten Alkylrest mit 12 bis 18 Kohlenstoffatomen. Beispiele hierfür sind Dodecyl, Tridecyl, Tetradecyl, Pentadecyl, Hexadecyl, Heptadecyl, Octadecyl und Stellungsisomere davon.

C₁₂-Alkyl steht für Dodecyl und dessen Stellungsisomere.

Die nachfolgenden Äußerungen zu bevorzugten Ausführungsformen, insbesondere zu bevorzugten Ausführungsformen der Verbindungen I, ihrer Verwendung und der sie enthaltenden Mittel gelten sowohl allein für sich genommen als auch in jeder denkbaren Kombination miteinander. Die Ausführungen gelten, soweit nichts Gegenteiliges erwähnt wird, sowohl für die erfindungsgemäße Verwendung als auch für die erfindungsgemäßen Wasch- und Reinigungsmittel.

Bei den erfindungsgemäß zum Einsatz kommenden Verbindungen I kann es sich um chemisch reine Substanzen oder um Gemische verschiedener Verbindungen I handeln. In der Regel wird es sich aufgrund ihres Herstellungsverfahrens und der darin eingesetzten Edukte, die technische Produkte oder Produktgemische sein können, um Gemische verschiedener Verbindungen I handeln, die sich beispielsweise in der Bedeutung der Variablen R, R', n, x und/oder y unterscheiden.

Vorzugsweise stehen R und R' unabhängig voneinander für C₈-C₁₆-Alkyl, besonders bevorzugt für C₈-C₁₄-Alkyl und insbesondere für C₁₀-C₁₄-Alkyl.

Bevorzugt stehen R und R' für einen linearen Alkylrest.

Bevorzugt haben R und R' die gleiche Bedeutung.

In einer speziellen Ausführungsform stehen R und R' für C₁₂-Alkyl, insbesondere für n-Dodecyl.

n steht vorzugsweise für eine Zahl von 15 bis 100, besonders bevorzugt von 20 bis 80, stärker bevorzugt von 25 bis 45 und insbesondere von 30 bis 40.

x und y stehen unabhängig voneinander vorzugsweise für 0 oder 1, besonders bevorzugt für 0.

Die Verbindungen I, worin x und y für 0 stehen, lassen sich durch Kondensation von Polyethylenglykolen (die mit OH-Gruppen terminiert sein müssen) mit Alkylmercaptoethanolderivaten der Formel R-S-CH₂CH₂-OH bzw. R'-S-CH₂CH₂-OH herstellen. Thioether I, in denen x und/oder y von 0 verschieden sind, lassen sich durch Oxidation der Thioether I, worin x und y für 0 stehen, herstellen.

Ein weiterer Gegenstand der vorliegenden Erfindung betrifft daher ein Verfahren zur Herstellung von Verbindungen I, bei dem man ein Polyethylenglykol der Formel

HO-[-CH₂CH₂-O-]ₙ₋₂-CH₂CH₂-OH

mit einem Alkylmercaptoethanolderivat der Formel R-S-CH₂CH₂-OH oder R'-S-CH₂CH₂-OH oder einem Gemisch davon (wenn R und R' verschieden sind) umsetzt.

Für den Fall, dass R und R' verschieden sind, kann die Umsetzung des Polyethylenglykols mit den verschiedenen Alkylmercaptoethanolderivaten der Formeln R-S-CH₂CH₂-OH und R'-S-CH₂CH₂-OH gleichzeitig oder nacheinander erfolgen. Um definierte Produkte zu erhalten, wird das Polyethylenglykol zunächst mit dem einen Mercaptoethanol umgesetzt, und erst nach beendeter Umsetzung mit diesem ersten Mercaptoethanol wird das erhaltene Produkt mit dem zweiten Mercaptoethanol zur Reaktion gebracht.

Diese Kondensation mit dem Mercaptoethanol erfolgt vorzugsweise unter saurer Katalyse. Geeignete Säuren sind beispielsweise p-Toluolsulfonsäure, Methansulfonsäure, Schwefelsäure, Phosphorsäure und saure lonentauscher. Während der Umsetzung wird vorzugsweise das gebildete Reaktionswasser entfernt, z.B. durch azeotrope Destillation oder Strippen mit einem Inertgas. Soll das Reaktionswasser durch azeotrope Destillation entfernt werden, so wird die Kondensation vorteilhafterweise in einem Lösemittel durchgeführt, das mit Wasser ein Minimumazeotrop bildet, wie Toluol oder die Xylole. Nach erfolgter Umsetzung kann das erhaltene Produktgemisch gewünschtenfalls neutralisiert werden.

Die Oxidation des Schwefelatoms im erhaltenen Thioether zum Monosulfoxid (x = 1, y = 0 oder umgekehrt), Disulfoxid (x, y = 1) bzw. Disulfon (x, y = 2) kann mittels üblicher Oxidationsmittel, wie Wasserstoffperoxid, Braunstein, ein Permanganat, m-Chlorperbenzoesäure oder ein Perchlorat erfolgen. Ob die Oxidation bis zum Mono- oder Disulfoxid oder bis zum Sulfon verläuft, kann u.a. durch die Wahl des Oxidationsmittels, dessen Konzentration und durch die Reaktionstemperatur bestimmt werden.

Die eingesetzten Polyethylenglykole sind kommerziell erhältlich. Sie lassen sich beispielsweise durch Polymerisation von Ethylenoxid bzw. durch Addition von Ethylenoxid an Ethylenglykol erhalten.

Die Alkylmercaptoethanolderivate sind ebenfalls kommerziell erhältlich. Sie lassen sich beispielsweise durch Addition von Thioethylenglykol an ein Alken erhalten.

Die Verbindungen I können in Form der bei ihrer Herstellung anfallenden Lösung erfindungsgemäß eingesetzt werden. Vorzugsweise werden sie jedoch mittels üblicher Verfahren isoliert und gewünschtenfalls gereinigt.

Die Verbindungen der Formel I sind prinzipiell in allen gängigen Wasch- und Reinigungsmitteln einsetzbar.

Unter Waschmitteln versteht man dabei im Rahmen der vorliegenden Erfindung solche Mittel, die zum Reinigen von flexiblen Materialien mit hoher Saugfähigkeit verwendet werden, z.B. von Materialien mit einem textilen Charakter, während man unter Reinigungsmitteln im Rahmen der vorliegenden Erfindung solche Mittel versteht, die zum Reinigen von Materialien mit geschlossener Oberfläche, d.h. mit einer Oberfläche, die keine oder nur wenige und kleine Poren hat und infolgedessen keine oder nur eine geringe Saugfähigkeit aufweist, eingesetzt werden.

Beispiele für flexible Materialien mit hoher Saugfähigkeit sind solche, die natürliche, synthetische oder halbsynthetische Fasermaterialien enthalten oder daraus bestehen und die demzufolge in der Regel zumindest teilweise einen textilen Charakter aufweisen. Die faserhaltigen oder aus Fasern bestehenden Materialien können prinzipiell in jeder im Gebrauch oder der Herstellung und Verarbeitung vorkommenden Form vorliegen. Beispielsweise können Fasern ungeordnet in Form von Flocke oder Haufwerk, geordnet in Form von Fäden, Garnen, Zwirnen, oder in Form von Flächengebilden wie Vliesen, Lodenstoffen oder Filz, Geweben, Gewirken in allen denkbaren Bindungsarten vorliegen. Bei den Fasern kann es sich um Rohfasern oder um Fasern in beliebigen Verarbeitungsstadien handeln. Beispiele sind natürliche Eiweiß- oder Zellulosefasern, wie Wolle, Seide, Baumwolle, Sisal, Hanf oder Kokosfasern, oder synthetische Fasern, wie beispielsweise Polyester-, Polyamid- oder Polyacrylnitrilfasern.

Beispiele für Materialien, die keine oder nur wenige und kleine Poren haben und keine oder nur eine geringe Saugfähigkeit aufweisen, sind Metall, Glas, Email oder Keramik. Typische Objekte aus diesen Materialien sind z.B. metallene Spülbecken, Bestecke, Glas- und Porzellangeschirr, Badewannen, Waschbecken, Kacheln, Fliesen, ausgehärtete Kunstharze, wie z.B. dekorative Melaminharzoberflächen auf Küchenmöbeln oder lackierte Metallflächen wie z.B. Kühlschränke und Autokarosserien, gedruckten Schaltungen (printed circuit boards), Mikrochips, versiegelte oder lackierte Hölzer, z.B. Parkett oder Wandverkleidungen, Fensterrahmen, Türen, Kunststoffbeläge wie Fußbodenbeläge aus PVC oder Hartgummi, oder Hart- oder Weichschaumstoffe mit weitgehend geschlossenen Oberflächen.

Beispiele für Reinigungsmittel, die die Verbindungen I enthalten, umfassen Geschirrspülmittel, wie Handgeschirrspülmittel oder Maschinengeschirrspülmittel (= Geschirrspülmittel für die Geschirrspülmaschine), Metallentfetter, Glasreiniger, Fußbodenreiniger, Allzweckreiniger, Hochdruckreiniger, Neutralreiniger, alkalische Reiniger, saure Reiniger, Spritzentfetter, Molkereireiniger, Großküchenreiniger, Apparatereiniger in der Industrie, insbesondere der chemischen Industrie, Reiniger für der Autowäsche und auch Haushalts-Allzweckreiniger.

Bevorzugt werden die Verbindungen I in Geschirrspülmitteln eingesetzt. Besonders bevorzugt werden sie in Maschinengeschirrspülmitteln eingesetzt. Bevorzugt sind hierunter Geschirrspülmittel, insbesondere Maschinengeschirrspülmittel, mit Klarspülfunktion.

Bevorzugt sind die die Wasch- und Reinigungsmittel, die die Verbindungen I enthalten, bei Raumtemperatur (20 °C) fest.

Bei den festen Wasch- und Reinigungsmitteln kann es sich um pulverförmige oder tablettenförmige Produkte ("Tabs") handeln. Vorzugsweise handelt es sich um tablettenförmige Produkte ("Tabs"). Besonders bevorzugt handelt es sich um tablettenförmige Geschirrspülmittel, insbesondere um tablettenförmige Maschinengeschirrspülmittel.

Bei tablettenförmigen Geschirrspülmitteln kann es sich um einfache Tabs oder auch um sogenannte "2 in 1"-, "3 in 1"-, "5 in 1"-, "7 in 1"-Produkte und dergleichen (multifunktionelle Produkte; allgemein gesprochen "x in 1"-Produkte mit x = ganze Zahl) handeln. Näheres zu diesen Formulierungen findet sich in Hermann G. Hauthal, G. Wagner (Hrsg.), Reinigungs- und Pflegemittel im Haushalt, Verlag für chemische Industrie, H. Ziolkowsky GmbH, Augsburg 2003, Kapitel 4.2, Seiten 161-184. "2 in 1"-Produkte enthalten neben den üblichen Bestandteilen von Maschinengeschirrspülmitteln zusätzlich einen Klarspüler. "3 in 1"-Produkte enthalten außerdem einen Wasserenthärter. "5 in 1"-Produkte enthalten in der Regel außerdem ein Glasschutzmittel und ein Spülkraftverstärkungsmittel. "7 in 1"-Produkte enthalten außerdem ein Edelstahlglanzmittel und ein Entkrustungsmittel.

Vorzugsweise werden die Verbindungen I in tablettenförmigen multifunktionellen Maschinengeschirrspülmitteln eingesetzt, wo sie die üblichen Klarspüler ganz oder teilweise ersetzen.

Die Verbindungen I haben sowohl eine Wirkung als Tensid als auch als Klarspülmittel. Daher betrifft die Erfindung auch die Verwendung der Verbindungen I als Tensid und/oder als Klarspülmittel. Insbesondere betrifft die Erfindung die Verwendung der Verbindungen I als Tensid mit Klarspüleffekt bzw. als Klarspültensid.

Die erfindungsgemäß verwendeten Verbindungen I zeichnen sich insbesondere durch eine hervorragende belagsinhibierende Wirkung beim Einsatz im Klarspülgang des maschinellen Geschirrspülers aus. Sie wirken dabei sowohl gegenüber anorganischen als auch gegenüber organischen Belägen inhibierend. Bei den anorganischen Belägen handelt es sich insbesondere um Calcium- und Magnesiumphosphat, -carbonat, -silikat und/oder -phosphonat, die aus den im Wasser enthaltenen Calcium und Magnesiumsalzen und den in üblichen Geschirrspülmitteln enthaltenen Buildern entstehen. Bei den organischen Belägen handelt es sich insbesondere um Schmutzbestandteile aus der Spülflotte, wie Eiweiß-, Stärke- und Fettbeläge. Die erfindungsgemäß verwendeten Verbindungen I sind auch gegenüber sogenannten Carry-Over-Belägen wirksam, die aus der Restwassermenge im Sumpf der Spülmaschine stammen und u. a. Geschirrspülmittelreste und eventuell auch noch Schmutzreste aus dem vorhergehenden Waschgang der Geschirrspülmaschine enthalten.

Ein weiterer Gegenstand der Erfindung sind Wasch- oder Reinigungsmittel, die wenigstens eine Verbindung der Formel I enthalten. Bezüglich geeigneter Wasch- oder Reinigungsmittel wird auf die obigen Ausführungen verwiesen.

Vorzugsweise handelt es sich bei den Wasch- und Reinigungsmitteln um Geschirrspülmittel, worunter Maschinengeschirrspülmittel bevorzugt sind. Insbesondere handelt es sich um Geschirrspülmittel, insbesondere Maschinengeschirrspülmittel, mit Klarspülfunktion.

Das erfindungsgemäße Wasch- und Reinigungsmittel ist bei Raumtemperatur (20 °C) vorzugsweise fest. Bezüglich geeigneter und bevorzugter fester Wasch- und Reinigungsmittel wird auf die vorstehenden Ausführungen verwiesen. Insbesondere sind die erfindungsgemäßen Wasch- und Reinigungsmittel tablettenförmige multifunktionelle Maschinengeschirrspülmittel. In diesen können die Verbindungen I im Klarspülkern enthalten sein; bevorzugt sind sie jedoch als Feststoff im tablettenförmigen Geschirrspülmittel enthalten.

Das erfindungsgemäße Geschirrspülmittel umfasst vorzugsweise folgende Bestandteile:
a) wenigstens eine Verbindung der Formel I;
b) wenigstens einen Builder (auch als Sequestrierungsmittel, Gerüststoff, Komplexbildner, Chelator, Chelatisierungsmittel oder Enthärter bezeichnet);
c) gegebenenfalls wenigstens ein Enzym; und
d) gegebenenfalls wenigstens ein Bleichmittel; und
e) gegebenenfalls wenigstens einen weiteren Zusatzstoff, der vorzugsweise ausgewählt ist unter Tensiden, die von a) verschieden sind, Basen, Korrosionsinhibitoren, Entschäumern, Farbstoffen, Duftstoffen, Füllstoffen, Tablettierhilfsmitteln, Desintegrationsmitteln, Verdickern, Löslichkeitsvermittlern, organischen Lösemitteln und Wasser.

Vorzugsweise sind diese Bestandteile in folgenden Mengenverhältnissen im erfindungsgemäßen Geschirrspülmittel enthalten:
a) wenigstens eine Verbindung der Formel I: von 0,1 bis 20 Gew.-%;
b) wenigstens ein Builder: von 5 bis 80 Gew.-%;
c) wenigstens ein Enzym: von 0 bis 8 Gew.-%;
d) wenigstens ein Bleichmittel: von 0 bis 30 Gew.-%; und
e) wenigstens ein weiterer Zusatzstoff: von 0 bis 50 Gew.-%.

Die Gew.-%-Angaben beziehen sich dabei auf das Gesamtgewicht des Geschirrspülmittels. Die Gewichtsmengen von a) bis e) ergänzen sich zu 100 Gew.-%.

Besonders bevorzugt umfasst das erfindungsgemäße Geschirrspülmittel wenigstens ein Enzym.

Besonders bevorzugt sind die oben genannten Bestandteile in folgenden Mengenverhältnissen im erfindungsgemäßen Geschirrspülmittel enthalten:
a) wenigstens eine Verbindung der Formel I: von 0,1 bis 10 Gew.-%;
b) wenigstens ein Builder: von 20 bis 80 Gew.-%;
c) wenigstens ein Enzym: von 0,1 bis 6 Gew.-%;
d) wenigstens ein Bleichmittel: von 0 bis 30 Gew.-%; und
e) wenigstens ein weiterer Zusatzstoff: von 0 bis 50 Gew.-%.

Stärker bevorzugt umfasst das erfindungsgemäße Geschirrspülmittel außerdem wenigstens ein Bleichmittel.

Stärker bevorzugt sind die oben genannten Bestandteile in folgenden Mengenverhältnissen im erfindungsgemäßen Geschirrspülmittel enthalten:
a) wenigstens eine Verbindung der Formel I: von 0,1 bis 10 Gew.-%;
b) wenigstens ein Builder: von 20 bis 80 Gew.-%;
c) wenigstens ein Enzym: von 0,1 bis 6 Gew.-%;
d) wenigstens ein Bleichmittel: von 5 bis 25 Gew.-%; und
e) wenigstens ein weiterer Zusatzstoff: von 0 bis 50 Gew.-%.

Bezüglich geeigneter und bevorzugter Verbindungen I wird auf die vorstehenden Ausführungen verwiesen.

Builder, die teilweise auch als Sequestrierungsmittel, Gerüststoff, Komplexbildner, Chelator, Chelatisierungsmittel oder Enthärter bezeichnet werden, binden Erdalkalimetalle und andere wasserlösliche Metallsalze, ohne zu präzipitieren. Sie helfen Schmutz aufzubrechen, dispergieren Schmutzteilen, helfen Schmutz abzulösen und haben teilweise eine eigene Waschwirkung. Außerdem halten sie, wenn sie fest sind und in pulverförmigen Formulierungen eingesetzt werden, das Pulver rieselfähig.

Geeignete Builder können sowohl organischer als auch anorganischer Natur sein. Beispiele sind Alumosilikate, Carbonate, Phosphate und Polyphosphate, Polycarbonsäuren, Polycarboxylate, Hydroxycarbonsäuren, Phosphonsäuren, z.B. Hydroxyalkylphosphonsäuren, Phosphonate, Aminopolycarbonsäuren und deren Salze und polymere carbonsäuregruppenhaltige Verbindungen und deren Salze.

Geeignete anorganische Builder sind beispielsweise kristalline oder amorphe Alumosilikate mit ionenaustauschenden Eigenschaften, wie Zeolithe. Verschiedene Typen von Zeolithen sind geeignet, insbesondere Zeolithe A, X, B, P, MAP und HS in ihrer Na-Form oder in Formen, in denen Na teilweise gegen andere Kationen wie Li, K, Ca, Mg oder Ammonium ausgetauscht ist. Geeignete Zeolithe sind beispielsweise in der US-A-4604224 beschrieben. Als Builder geeignete kristalline Silikate sind beispielsweise Disilikate oder Schichtsilikate, z.B. 5-Na₂Si₂O₅ oder B-Na₂Si₂O₅ (SKS 6 bzw. SKS 7). Die Silikate können in Form ihrer Alkalimetall-, Erdalkalimetall- oder Ammoniumsalze eingesetzt werden, vorzugsweise als Na-, Li- und Mg-Silikate. Amorphe Silikate wie beispielsweise Natriummetasilikat, welches eine polymere Struktur aufweist, oder amorphes Disilikat (Britesil® H 20 Hersteller: Akzo) sind ebenfalls verwendbar. Bevorzugt ist hierunter Natriumdisilikat.

Geeignete anorganische Buildersubstanzen auf Carbonat-Basis sind Carbonate und Hydrogencarbonate. Diese können in Form ihrer Alkalimetall-, Erdalkalimetall- oder Ammoniumsalze eingesetzt werden. Vorzugsweise werden Na-, Li- und Mg-Carbonate bzw. -Hydrogencarbonate, insbesondere Natriumcarbonat und/oder Natriumhydrogencarbonat, eingesetzt.

Übliche, als anorganische Builder eingesetzte Phosphate sind Alkalimetallorthophosphate und/oder -polyphosphate wie z.B. Pentanatriumtriphosphat.

Geeignete organische Builder sind beispielsweise C₄-C₃₀-Di-, -Tri- und -Tetracarbonsäuren, wie z.B. Bernsteinsäure, Propantricarbonsäure, Butantetracarbonsäure, Cyclopentantetracarbonsäure und Alkyl- und Alkenylbernsteinsäuren mit C₂-C₂₀-Alkyl- bzw. -Alkenyl-Resten.

Geeignete organische Builder sind weiterhin Hydroxycarbonsäuren und Polyhydroxycarbonsäuren (Zuckersäuren). Dazu zählen C₄-C₂₀-Hydroxycarbonsäuren wie z.B. Äpfelsäure, Weinsäure, Gluconsäure, Schleimsäure, Milchsäure, Glutarsäure, Citronensäure, Tartronsäure, Glucoheptonsäure, Lactobionsäure und Saccharosemono-, -di- und -tricarbonsäure. Bevorzugt ist hierunter Citronensäure und ihre Salze.

Geeignete organische Builder sind weiterhin Phosphonsäuren wie z.B. Hydroxyalkylphosphonsäuren, Aminophosphonsäuren und die Salze davon. Dazu zählen z.B. Phosphonobutantricarbonsäure, Aminotris-methylenphosphonsäure, Ethylendiamintetraethylenphosphonsäure, Hexamethylendiamintetramethylenphosphonsäure, Diethylentriamin-pentamethylenphosphonsäure, Morpholino-methandiphosphonsäure, 1-Hydroxy-C₁- bis C₁₀-alkyl-1,1-diphosphonsäuren wie 1-Hydroxyethan-1,1-diphosphonsäure. Bevorzugt ist hierunter 1-Hydroxyethan-1,1-diphosphonsäure und deren Salze.

Geeignete organische Builder sind weiterhin Aminopolycarbonsäuren, wie Nitrilotriessigsäure (NTA), Nitrilomonoessigdipropionsäure, Nitrilotripropionsäure, β-Alanindiessigsäure (β-ADA), Ethylendiamintetraessigsäure (EDTA), Diethylentriaminpentaessigsäure, 1,3-Propylendiamintetraessigsäure, 1,2-Propylendiamintetraessigsäure, N-(Alkyl)-ethylendiamintriessigsäure, N-(Hydroxyalkyl)-ethylendiamintriessigsäure, Ethylendiamintriessigsäure, Cyclohexylen-1,2-diamintetraessigsäure, Iminodibernsteinsäure, Ethylendiamindibernsteinsäure, Serindiessigsäure, Isoserindiessigsäure, L-Asparagindiessigsäure, L-Glutamindiessigsäure, Methylglycindiessigsäure (MGDA) und die Salze der zuvorgenannten Aminopolycarbonsäuren. Bevorzugt sind hierunter L-Glutamindiessigsäure, Methylglycindiessigsäure und deren Salze.

Geeignete organische Builder sind weiterhin polymere carbonsäuregruppenhaltige Verbindungen, wie Acrylsäure-Homopolymere. Diese weisen vorzugsweise ein zahlenmittleres Molekulargewicht im Bereich von 800 bis 70000 g/mol, besonders bevorzugt von 900 bis 50000 g/mol, insbesondere von 1000 bis 20000 g/mol, speziell 1000 bis 10000 g/mol, auf. Der Begriff Acrylsäure-Homopolymer umfasst dabei auch Polymere, in denen die Carbonsäuregruppen teilweise oder vollständig neutralisiert vorliegen. Dazu zählen Acrylsäure-Homopolymere, in denen die Carbonsäuregruppen teilweise oder vollständig in Form von Alkalimetallsalzen oder Ammoniumsalzen vorliegen. Bevorzugt sind Acrylsäure-Homopolymere, in denen die Carbonsäuregruppen protoniert sind oder in denen die Carbonsäuregruppen teilweise oder vollständig in Form von Natriumsalzen vorliegen.

Geeignete polymere carbonsäuregruppenhaltige Verbindungen sind auch Oligomaleinsäuren, wie sie beispielsweise in EP-A 451 508 und EP-A 396 303 beschrieben sind.

Geeignete polymere carbonsäuregruppenhaltige Verbindungen sind auch Terpolymere ungesättigter C₄-C₈-Dicarbonsäuren, wobei als Comonomere monoethylenisch ungesättigte Monomere aus der unten angegebenen Gruppe (i) in Mengen von bis zu 95 Gew.-%, aus der Gruppe (ii) in Mengen von bis zu 60 Gew.-% und aus der Gruppe (iii) in Mengen von bis zu 20 Gew.-% einpolymerisiert sein können. Als ungesättigte C₄-C₈-Dicarbonsäuren sind hierbei beispielsweise Maleinsäure, Fumarsäure, Itaconsäure und Citraconsäure geeignet. Bevorzugt wird Maleinsäure. Die Gruppe (i) umfasst monoethylenisch ungesättigte C₃-C₈-Monocarbonsäuren wie z.B. Acrylsäure, Methacrylsäure, Crotonsäure und Vinylessigsäure. Bevorzugt werden aus der Gruppe (i) Acrylsäure und Methacrylsäure eingesetzt. Die Gruppe (ii) umfasst monoethylenisch ungesättigte C₂-C₂₂-Olefine, Vinylalkylether mit C₁-C₈-Alkylgruppen, Styrol, Vinylester von C₁-C₈-Carbonsäuren, (Meth)acrylamid und Vinylpyrrolidon. Bevorzugt werden aus der Gruppe (ii) C₂-C₆-Olefine, Vinylalkylether mit C₁-C₄-Alkylgruppen, Vinylacetat und Vinylpropionat eingesetzt. Falls die Polymeren der Gruppe (ii) Vinylester einpolymerisiert enthalten, können diese auch teilweise oder vollständig zu Vinylalkohol-Struktureinheiten hydrolysiert vorliegen. Geeignete Co- und Terpolymere sind beispielsweise aus US-A 3887806 sowie DE-A 4313909 bekannt. Die Gruppe (iii) umfasst (Meth)acrylester von C₁-C₈-Alkoholen, (Meth)acrylnitril, (Meth)acrylamide von C₁-C₈-Aminen, N-Vinylformamid und N-Vinylimidazol.

Geeignete polymere carbonsäuregruppenhaltige Verbindungen sind auch Homopolymere der monoethylenisch ungesättigten C₃-C₈-Monocarbonsäuren wie z.B. Acrylsäure, Methacrylsäure, Crotonsäure und Vinylessigsäure, insbesondere der Acrylsäure und Methacrylsäure, Copolymere von Dicarbonsäuren, wie z.B. Copolymere von Maleinsäure und Acrylsäure im Gewichtsverhältnis 10:90 bis 95:5, besonders bevorzugt solche im Gewichtsverhältnis 30:70 bis 90:10 mit Molmassen von 1000 bis 150000; Terpolymere aus Maleinsäure, Acrylsäure und einem Vinylester einer C₁-C₃- Carbonsäure im Gewichtsverhältnis 10 (Maleinsäure) :90 (Acrylsäure + Vinylester) bis 95 (Maleinsäure) :10 (Acrylsäure + Vinylester), wobei das Gew.-Verhältnis von Acrylsäure zum Vinylester im Bereich von 30:70 bis 70:30 variieren kann; Copolymere von Maleinsäure mit C₂-C₈-Olefinen im Molverhältnis 40:60 bis 80:20, wobei Copolymere von Maleinsäure mit Ethylen, Propylen oder Isobuten im Molverhältnis 50:50 besonders bevorzugt sind.

Geeignete polymere carbonsäuregruppenhaltige Verbindungen sind weiterhin Copolymere von 50 bis 98 Gew.-% ethylenisch ungesättigter schwacher Carbonsäuren mit 2 bis 50 Gew.-% ethylenisch ungesättigter Sulfonsäuren, wie sie beispielsweise in der EP-A-0877002 beschrieben sind. Geeignete schwache ethylenisch ungesättigte Carbonsäuren sind insbesondere C₃-C₆-Monocarbonsäuren, wie Acrylsäure und Methacrylsäure. Geeignete ethylenisch ungesättigte Sulfonsäuren sind 2-Acetylamidomethyl-1-propansulfonsäure, 2-Methacrylsäureamido-2-methyl-1-propansulfonsäure, 2-Methacrylamindo-2-hydroxypropansulfonsäure, Allylsulfonsäure, Methallylsulfonsäure, Allyloxybenzolsulfonsäure, Methallyloxybenzolsulfonsäure, 2-Hydroxy-3-(2-propenyloxy)propansulfonsäure, 2-Methyl-2-propen-1-sulfonsäure, Styrolsulfonsäure, Vinylsulfonsäure, 3-Sulfopropylacrylat, 3-Sulfopropylmethacrylat, Sulfomethylacrylamid, Sulfomethylmethacrylamid und Salze dieser Säuren. Die Copolymere können weiterhin 0 bis 30 Gew.-% ethylenisch ungesättigter C₄-C₈-Dicarbonsäuren, wie Maleinsäure, sowie 0 bis 30 Gew.-% wenigstens eines Monomers, das mit den zuvor genannten Monomeren copolymerisierbar ist, einpolymerisiert enthalten. Bei letzterem handelt es sich beispielsweise um C₁-C₄-Alkylester von (Meth)Acrylsäure, C₁-C₄-Hydroxyalkylester von (Meth)Acrylsäure, Acrylamid, Alkyl-substituiertes Acrylamid, N,N-Dialkyl-substituiertes Acrylamid, Vinylphosphonsäure, Vinylacetat, Allylalkohole, sulfonierte Allylalkohole, Styrol und andere Vinylaromaten, Acrylonitril, N-Vinylpyrrolidon, N-Vinylformamid, N-Vinylimidazol oder N-Vinylpyridin. Das gewichtsmittlere Molekulargewicht dieser Copolymere liegt im Bereich von 3000 bis 50000. Besonders geeignet sind Copolymere mit etwa 77 Gew.-% wenigstens einer ethylenisch ungesättigten C₃-C₆-Monocarbonsäure und etwa 23 Gew.-% wenigstens einer ethylenisch ungesättigten Sulfonsäure.

Pfropfpolymere ungesättigter Carbonsäuren auf niedermolekulare Kohlenhydrate oder hydrierte Kohlenhydrate, vgl. US-A 5227446, DE-A 4415623 und DE-A 4313909, eignen sich ebenfalls. Geeignete ungesättigte Carbonsäuren sind hierbei beispielsweise Maleinsäure, Fumarsäure, Itaconsäure, Citraconsäure, Acrylsäure, Methacrylsäure, Crotonsäure und Vinylessigsäure sowie Mischungen aus Acrylsäure und Maleinsäure, die in Mengen von 40 bis 95 Gew.-%, bezogen auf die zu pfropfende Komponente, aufgepfropft werden. Zur Modifizierung können zusätzlich bis zu 30 Gew.-%, bezogen auf die zu pfropfende Komponente, weitere monoethylenisch ungesättigte Monomere einpolymerisiert vorliegen. Geeignete modifizierende Monomere sind die oben genannten Monomere der Gruppen (ii) und (iii). Als Pfropfgrundlage sind abgebaute Polysaccharide wie z.B. sauer oder enzymatisch abgebaute Stärken, Inuline oder Zellulose, Eiweisshydrolysate und reduzierte (hydrierte oder hydrierend aminierte) abgebaute Polysaccharide wie z.B. Mannit, Sorbit, Aminosorbit und N-Alkylglucamin geeignet sowie auch Polyalkylenglykole mit Molmassen mit bis zu M_{w} = 5000 wie z. B. Polyethylenglykole, Ethylenoxid/Propylenoxidbzw. Ethylenoxid/Butylenoxid bzw. Ethylenoxid/Propylenoxid/Butylenoxid-Blockcopolymere und alkoxylierte ein- oder mehrwertige C₁-C₂₂-Alkohole.(vgl. US-A-5756456).

Ebenfalls geeignet sind Polyglyoxylsäuren, wie sie beispielsweise in EP-B-001004, US-A-5399286, DE-A-4106355 und EP-A-656914 beschrieben sind. Die Endgruppen der Polyglyoxylsäuren können unterschiedliche Strukturen aufweisen.

Weiterhin sind Polyamidocarbonsäuren und modifizierte Polyamidocarbonsäuren geeignet; diese sind beispielsweise aus EP-A-454126, EP-B-511037, WO-A94/01486 und EP-A-581452 bekannt.

Auch Polyasparaginsäuren oder Cokondensate der Asparaginsäure mit weiteren Aminosäuren, C₄-C₂₅-Mono- oder -Dicarbonsäuren und/oder C₄-C₂₅-Mono- oder - Diaminen können als polymere carbonsäuregruppenhaltige Verbindungen eingesetzt werden. Besonders bevorzugt werden in phosphorhaltigen Säuren hergestellte, mit C₆-C₂₂-Mono- oder -Dicarbonsäuren bzw. mit C₆-C₂₂-Mono- oder -Diaminen modifizierte Polyasparaginsäuren eingesetzt.

Unter den polymeren carbonsäuregruppenhaltigen Verbindungen sind Polyacrylsäuren auch in teilweise oder vollständig neutralisierter Form, bevorzugt.

Als organische Builder eignen sich weiterhin Iminodibernsteinsäure, Oxydibernsteinsäure, Aminopolycarboxylate, Alkylpolyaminocarboxylate, Aminopolyalkylenphosphonate, Polyglutamate, hydrophob modifizierte Citronensäure wie z.B. Agaricinsäure, Poly-[alpha]-hydroxyacrylsäure, N-Acylethylendiamintriacetate wie Lauroylethylendiamintriacetat und Alkylamide der Ethylendiamintetraessigsäure wie EDTA-Talgamid.

Weiterhin können auch oxidierte Stärken als organische Builder verwendet werden.

Bevorzugt wird als Komponente b) ein Gemisch verschiedener Builder eingesetzt.

Bevorzugt enthält das Gemisch verschiedener Builder wenigstens zwei der folgenden Bestandteile: wenigstens ein Carbonat (z.B. Natriumcarbonat), wenigstens ein Silikat (z.B. Natriumdisilkat), wenigstens eine polymere carbonsäuregruppenhaltige Verbindung oder wenigstens eine polymere carbonsäuregruppenhaltige Verbindung, in der die Carbonsäuregruppen teilweise oder vollständig neutralisiert vorliegen (z.B. Polyacrylsäure), wenigstens eine (Poly)Hydroxycarbonsäure oder ein Salz davon (z.B. Citronensäure oder ein Citrat), wenigstens eine Aminopolycarbonsäure oder ein Salz davon (z.B. Methylglycindiessigsäure oder ein Salz davon, z.B. ein Natriumsalz davon), wenigstens eine Phosphonsäure (z.B. 1-Hydroxyethan1-(1,1-diphosphonsäure); HEDP), wenigstens ein Phosphat. Besonders bevorzugt enthält das Gemisch wenigstens ein Carbonat, wenigstens ein Silikat und wenigstens eine polymere gegebenenfalls (teil)neutralisierte carbonsäuregruppenhaltige Verbindung sowie optional wenigstens einen der folgenden Bestandteile: wenigstens eine (Poly)Hydroxycarbonsäure oder ein Salz davon, wenigstens eine Phosphonsäure, wenigstens ein Phosphat. Speziell enthält das Gemisch wenigstens ein Carbonat, wenigstens ein Silikat, wenigstens eine polymere gegebenenfalls (teil)neutralisierte carbonsäuregruppenhaltige Verbindung, wenigstens eine (Poly)Hydroxycarbonsäure oder ein Salz davon und wenigstens eine Phosphonsäure, sowie optional wenigstens ein Phosphat.

In einem solchen Gemisch sind die Bestandteile vorzugsweise in folgenden Mengen enthalten:
b1) wenigstens ein Carbonat: 10 bis 50 Gew.-%;
b2) wenigstens ein Silikat: 1 bis 10 Gew.-%;
b3) wenigstens eine polymere gegebenenfalls (teil)neutralisierte carbonsäuregruppenhaltige Verbindung: 5 bis 20 Gew.-%;
b4) wenigstens eine (Poly)Hydroxycarbonsäure oder ein Salz davon: 0 bis 50 Gew.-%;
b5) wenigstens eine Aminopolycarbonsäure oder ein Salz davon: 0 bis 60 Gew.-%; b6) wenigstens eine Phosphonsäure: 0,2 bis 1 Gew.-%;
b7) wenigstens ein Phosphat: 0 bis 60 Gew.-%.

Die Gew.-%-Angaben beziehen sich dabei auf das Gesamtgewicht des Builders. Die Gewichtsmengen von b1) bis b7) ergänzen sich zu 100 Gew.-%.

Die Enzyme sind vorzugsweise ausgewählt unter Hydrolasen, wie Proteasen, Esterasen, Glucosidasen, Lipasen, Amylasen, Cellulasen, Mannanasen, anderen Glykosylhydrolasen und Gemischen der zuvor genannten Enzyme. Alle diese Hydrolasen tragen zur Schmutzauflösung und -entfernung von protein-, fett- oder stärkehaltigen Verschmutzungen bei. Zur Bleiche können auch Oxireduktasen eingesetzt werden. Besonders gut geeignet sind aus Bakterienstämmen oder Pilzen wie Bacillus subtilis, Bacillus licheniformis, Streptomyceus griseus und Humicola insolens gewonnene enzymatische Wirkstoffe.

Geeignete Hydrolasen sind z.B. α-Glucosidasen (EC-Nummer 3.2.1.20), Proteasen (Ovozyme® (von Novozymes); EC-Nummer 3.2.1.20), Amylasen [Purastar® (von Genencor), Termamyl® (von Novozymes), Stainzyme® (von Novozymes), Duramyl® (von Novozymes)], Mannanasen [Purabrite® (von Genencor), Mannastar® (von Genencor), Mannaway® (von Novozymes)] und Cellulasen [Carezyme® (von Novozymes), Celluzyme® (von Novozymes), Endolase®, Puradax® (von Genencor)]. Zu den geeigneten Amylasen zählen insbesondere α-Amylasen (EC-Nummer 3.2.1.1), Iso-Amylasen, Pullulanasen und Pektinasen. Als Cellulasen werden vorzugsweise Cellobiohydrolasen, Endoglucanasen und β-Glucosidasen, die auch Cellobiasen genannt werden, bzw. Mischungen aus diesen eingesetzt. Da sich verschiedene Cellulase-Typen durch ihre CMCase- und Avicelase-Aktivitäten unterscheiden, können durch gezielte Mischungen der Cellulasen die gewünschten Aktivitäten eingestellt werden.

Geeignete Lipasen sind Esterasen, wie Lipex und Lipolase. Beispiele für lipolytisch wirkende Enzyme sind die bekannten Cutinasen.

Auch Peroxidasen oder Oxidasen haben sich in einigen Fällen als geeignet erwiesen.

Vorzugsweise enthält das erfindungsgemäße Geschirrspülmittel wenigstens eine Protease und/oder Amylase.

Vorzugsweise enthält das erfindungsgemäße Geschirrspülmittel eine Enzymmischung. Bevorzugt sind beispielsweise Enzymmischungen, die folgende Enzyme enthalten oder aus ihnen bestehen:
- Protease und Amylase,
- Protease und Lipase (bzw. lipolytisch wirkenden Enzymen),
- Protease und Cellulase,
- Amylase, Cellulase und Lipase (bzw. lipolytisch wirkenden Enzymen),
- Protease, Amylase und Lipase (bzw. lipolytisch wirkenden Enzymen)
- Protease, Lipase (bzw. lipolytisch wirkenden Enzymen) und Cellulase.

Besonders bevorzugt sind Protease und/oder Amylase-haltige Mischungen. Bevorzugt als Proteasen in den zuvor genannten Mischungen sind Proteasen vom Subtilisin-Typ (Savinase, etc.; EC-Nummer 3.4.21.62).

Die Enzyme können an Trägerstoffe adsorbiert sein, um sie gegen vorzeitige Zersetzung zu schützen.

Gegebenenfalls kann das erfindungsgemäße Wasch- und Reinigungsmittel noch Enzymstabilisatoren, z.B. Calciumpropionat, Natriumformiat oder Borsäuren oder deren Salze, und/oder Oxidationsverhinderer enthalten.

Bei den Bleichmitteln d) handelt es sich vorzugsweise um Bleichsysteme, die neben Bleichmitteln gegebenenfalls noch Bleichaktivatoren, Bleichkatalysatoren und/oder Bleichstabilisatoren enthalten.

Geeignete Bleichmittel sind beispielsweise Percarbonsäuren, z.B. Diperoxododecandicarbonsäure, Phthalimidopercapronsäure oder Monoperoxophthalsäure oder-terephthalsäure, Salze der Percarbonsäuren, z.B. Natriumpercarbonat, Addukte von Wasserstoffperoxid an anorganische Salze, z.B. Natriumperborat-Monohydrat, Natriumperborat-Tetrahydrat, Natriumcarbonat-Perhydrat oder Natriumphosphat-Perhydrat, Addukte von Wasserstoffperoxid an organische Verbindungen, z.B. Harnstoff-Perhydrat, oder von anorganischen Peroxosalzen, z.B. Alkalimetallpersulfaten, oder -peroxodisulfaten.

Als Bleichaktivatoren eignen sich beispielsweise polyacylierte Zucker, z.B. Pentaacetylglucose; Acyloxybenzolsulfonsäuren und deren Alkali- und Erdalkaiimetallsalze, z.B. Natrium-p-nonanoyloxybenzolsulfonat oder Natrium-p-benzoyloxybenzolsulfonat; - N,N-diacylierte und N,N,N',N'-tetraacylierte Amine, z.B. N,N,N',N'-Tetraacetylmethylendiamin und -ethylendiamin (TAED), N,N-Diacetylanilin, N,N-Diacetyl-ptoluidin oder 1,3-diacylierte Hydantoine wie 1,3-Diacetyl-5,5-dimethylhydantoin; N-Alkyl-N-sulfonylcarbonamide, z.B. N-Methyl-N-mesylacetamid oder N-MethylN-mesylbenzamid; N-acylierte cyclische Hydrazide, acylierte Triazole oder Urazole, z.B. Monoacetylmaleinsäurehydrazid; O,N,N-trisubstituierte Hydroxylamine, z.B. O-Benzoyl-N,N-succinylhydroxylamin, O-Acetyl-N,N-succinylhydroxylamin oder O,N,N-Triacetylhydroxylamin; N,N'-Diacylsulfurylamide, z.B. N,N'-Dimethyl-N,N'-diacetylsulfurylamid oder N,N'- Diethyl-N,N'-dipropionylsulfurylamid; acylierte Lactame wie beispielsweise Acetylcaprolactam, Octanoylcaprolactam, Benzoylcaprolactam oder Carbonylbiscaprolactam; Anthranilderivate wie z.B. 2-Methylanthranil oder 2-Phenylanthranil; Triacylcyanurate, z.B. Triacetylcyanurat oder Tribenzoylcyanurat; Oximester und Bisoximester wie z.B. O-Acetylacetonoxim oder Bisisopropyliminocarbonat; Carbonsäureanhydride, z.B. Essigsäureanhydrid, Benzoesäureanhydrid, m-Chlorbenzoesäureanhydrid oder Phthalsäureanhydrid; Enolester wie z.B. Isopropenylacetat; 1,3-Diacyl-4,5-diacyloxy-imidazoline, z.B. 1,3-Diacetyl-4,5-diacetoxyimidazolin; Tetraacetylglycoluril und Tetrapropionylglycoluril; diacylierte 2,5-Diketopiperazine, z.B. 1,4-Diacetyl-2,5-diketopiperazin; ammoniumsubstituierte Nitrile wie z.B. N-Methylmorpholiniumacetonitrilmethylsulfat; Acylierungsprodukte von Propylendiharnstoff und 2,2-Dimethyl-propylendihamstoff, z.B. Tetraacetylpropylendihamstoff; α-Acyloxypolyacylmalonamide, z.B. α-Acetoxy-N,N'-diacetylmalonamid; Diacyldioxohexahydro-1 ,3,5-triazine, z.B. 1,5-Diacetyl-2,4-dioxohexahydro1,3,5-triazin; Benz-(4H)1,3-oxazin-4-one mit Alkylresten, z.B. Methyl, oder aromatischen Resten z.B. Phenyl, in der 2-Position.

Ein Bleichsystem aus Bleichmitteln und Bleichaktivatoren kann gegebenenfalls noch Bleichkatalysatoren enthalten. Geeignete Bleichkatalysatoren sind beispielsweise quaternierte Imine und Sulfonimine, die beispielsweise in US-A 5 360 569 und EP-A453 003 beschrieben sind. Besonders wirksame Bleichkatalysatoren sind Mangankomplexe, die beispielsweise in der WO-A 94/21777 beschrieben sind. Solche Verbindungen werden im Falle ihres Einsatzes in den Wasch- und Reinigungsmitteln höchstens in Mengen bis 1 ,5 Gew.-%, insbesondere bis 0,5 Gew.-%, im Falle von sehr aktiven Mangankomplexen in Mengen bis zu 0,1 Gew.-%, eingearbeitet. Neben dem beschriebenen Bleichsystem aus Bleichmitteln, Bleichaktivatoren und gegebenenfalls Bleichkatalysatoren ist für die erfindungsgemäßen Wasch- und Reinigungsmittel auch die Verwendung von Systemen mit enzymatischer Peroxidfreisetzung oder von photoaktivierten Bleichsystemen möglich.

Tenside aus der Gruppe e), die von Komponente a) verschieden sind, können kationisch, anionisch, zwitterionisch oder nichtionisch sein.

Geeignete nichtionische Tenside sind beispielsweise alkoxylierte, vorteilhafterweise ethoxylierte, insbesondere primäre Alkohole mit vorzugsweise 8 bis 18 C-Atomen und durchschnittlich 1 bis 20, vorzugsweise 1 bis 12 Mol Ethylenoxid (EO) pro Mol Alkohol, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, zum Beispiel aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C₁₂-C₁₄-Alkohole mit 3 EO, 4 EO oder 7 EO, C₉-C₁₁-Alkohol mit 7 EO, C₁₃-C₁₅-Alkohole mit 3 EO, 5 EO, 7 EO oder 8 EO, C₁₂-C₁₈-Alkohole mit 3 EO, 5 EO oder 7 EO und Mischungen aus diesen, wie Mischungen aus C₁₂-C₁₄-Alkohol mit 3 EO und C₁₂-C₁₈-Alkohol mit 7 EO. Die angegebenen Ethoxylierungsgrade stellen statistische Mittelwerte dar, die für ein spezielles Produkt eine ganze oder eine gebrochene Zahl sein können. Geeignet sind auch Alkoholethoxylate, die eine eingeengte Homologenverteilung aufweisen (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO oder 30 EO. Auch nichtionische Tenside, die EO- und PO-Gruppen zusammen im Molekül enthalten, sind einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere. Selbstverständlich sind auch gemischt alkoxylierte Niotenside einsetzbar, in denen EO- und PO-Einheiten nicht blockweise, sondern statistisch verteilt sind. Solche Produkte sind durch gleichzeitige Einwirkung von Ethylen- und Propylenoxid auf Fettalkohole erhältlich.

Außerdem können als weitere nichtionische Tenside auch Alkylglykoside der allgemeinen Formel (1)

R^{a}O(G)_{y} (1)

eingesetzt werden, worin R^{a} für einen primären geradkettigen oder methylverzweigten, insbesondere in 2-Stellung methylverzweigten aliphatischen Rest mit 8 bis 22, vorzugsweise 12 bis 18 C-Atomen steht und G für eine Glykosideinheit mit 5 oder 6 C-Atomen, vorzugsweise für Glucose, steht. Der Oligomerisierungsgrad y, der die Verteilung von Monoglykosiden und Oligoglykosiden angibt, ist eine beliebige Zahl zwischen 1 und 10; vorzugsweise liegt y bei 1,2 bis 1,4.

Eine weitere Klasse geeigneter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester, wie sie beispielsweise in der japanischen Patentanmeldung JP 58/217598 beschrieben sind oder die vorzugsweise nach dem in der internationalen Patentanmeldung WO-A-90/13533 beschriebenen Verfahren hergestellt werden.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Weitere geeignete Tenside sind Polyhydroxyfettsäureamide der Formel (2), 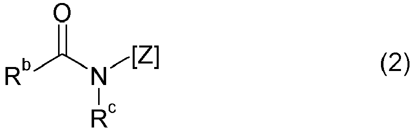 worin R^{b}C(=O) für einen aliphatischen Acylrest mit 6 bis 22 Kohlenstoffatomen, R^{c} für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (3) 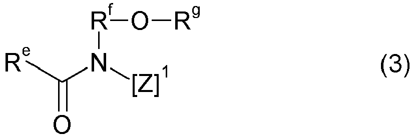 worin R^{e} für einen linearen oder verzweigten Alkyl- oder Alkenylrest mit 7 bis 12 Kohlenstoffatomen, R^{f} für einen linearen, verzweigten oder cyclischen Alkylenrest mit 2 bis 8 Kohlenstoffatomen oder einen Arylenrest mit 6 bis 8 Kohlenstoffatomen und R^{g} für einen linearen, verzweigten oder cyclischen Alkylrest oder einen Arylrest oder einen Oxy-Alkylrest mit 1 bis 8 Kohlenstoffatomen steht, wobei C₁-C₄-Alkyl- oder Phenylreste bevorzugt sind, und [Z]¹ für einen linearen Polyhydroxyalkylrest steht, dessen Alkylkette mit mindestens zwei Hydroxylgruppen substituiert ist, oder alkoxylierte, vorzugsweise ethoxylierte oder propoxylierte Derivate dieses Restes. [Z]¹ wird vorzugsweise durch reduktive Aminierung eines Zuckers erhalten, beispielsweise Glucose, Fructose, Maltose, Lactose, Galactose, Mannose oder Xylose. Die N-Alkoxy- oder N-Aryloxy-substituierten Verbindungen können dann beispielweise gemäß WO-A-95/07331 durch Umsetzung mit Fettsäuremethylestern in Gegenwart eines Alkoxids als Katalysator in die gewünschten Polyhydroxyfettsäureamide überführt werden.

Geeignete anionische Tenside sind beispielsweise solche vom Typ der Sulfonate und Sulfate. Als Tenside vom Sulfonat-Typ kommen dabei vorzugsweise C₉-C₁₃-Alkylbenzolsulfonate, Olefinsulfonate, d.h. Gemische aus Alken- und Hydroxyalkansulfonaten sowie Disulfonaten, wie man sie beispielsweise aus C₁₂-C₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht. Geeignet sind auch Alkansulfonate, die aus C₁₂-C₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), zum Beispiel die α-sulfonierten Methylester der hydrierten Kokos-, Palmkern- oder Talgfettsäuren geeignet.

Weitere geeignete Aniontenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monoglycerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂-C₁₆-Alkylsulfate und C₁₂-C₁₅-Alkylsulfate sowie C₁₄-C₁₅-Alkylsulfate bevorzugt. Auch 2,3-Alkylsulfate, welche beispielsweise gemäß den US-Patentschriften 3,234,258 oder 5,075,041 hergestellt werden und als Handelsprodukte der Shell Oil Company unter dem Namen DAN® erhalten werden können, sind geeignete Aniontenside.

Auch die Schwefelsäuremonoester der mit 1 bis 6 Mol Ethylenoxid ethoxylierten geradkettigen oder verzweigten C₇-C₂₁-Alkohole, wie 2-Methyl-verzweigte C₉-C₁₁-Alkohole mit im Durchschnitt 3,5 Mol Ethylenoxid (EO) oder C₁₂-C₁₈-Fettalkohole mit 1 bis 4 EO, sind geeignet. Sie werden in Reinigungsmitteln aufgrund ihres hohen Schaumverhaltens nur in relativ geringen Mengen, beispielsweise in Mengen von 1 bis 5 Gew.-%, eingesetzt.

Weitere geeignete Aniontenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxylierten Fettalkoholen darstellen. Bevorzugte Sulfosuccinate enthalten C₈-C₁₈- Fettalkoholreste oder Mischungen aus diesen. Insbesondere bevorzugte Sulfosuccinate enthalten einen Fettalkoholrest, der sich von ethoxylierten Fettalkoholen ableitet. Dabei sind wiederum Sulfosuccinate, deren Fettalkohol-Reste sich von ethoxylierten Fettalkoholen mit engerr Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen.

Geeignete anionische Tenside sind außerdem Seifen. Geeignet sind gesättigte und ungesättigte Fettsäureseifen, wie die Salze der Laurinsäure, Myristinsäure, Palmitinsäure, Stearinsäure, (hydrierten) Erucasäure und Behensäure sowie insbesondere aus natürlichen Fettsäuren, zum Beispiel Kokos-, Palmkern-, Olivenöl- oder Talgfettsäuren, abgeleitete Seifengemische.

Die anionischen Tenside einschließlich der Seifen können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze vor.

Kationische Tenside sind beispielsweise Ammoniumsalze, wie C₈-C₁₆-Dialkyldimethylammoniumhalogenide, Dialkoxydimethylammoniumhalogenide oder Imidazoliniumsalze mit langkettigem Alkylrest.

Amphotere Tenside sind beispielsweise Derivate von sekundären oder tertiären Aminen wie z.B. C₆-C₁₈-Alkylbetaine oder C₆-C₁₅-Alkylsulfobetaine oder Aminoxide wie Alkyldimethylaminoxide.

In Komponente e) enthaltene Lösemittel stammen beispielsweise aus der Gruppe ein- oder mehrwertigen Alkohole, Alkanolamine oder Glykolether. Vorzugsweise sind sie ausgewählt unter Ethanol, n- oder i-Propanol, Butanolen, Glykol, Propan- oder Butandiol, Glycerin, Diglykol, Propyl- oder Butyldiglykol, Hexylenglycol, Ethylenglykolmethylether, Ethylenglykolethylether, Ethylenglykolpropylether, Ethylenglykolmono-n-butylether, Diethylenglykol-methylether, Diethylenglykolethylether, Propylenglykolmethyl-, -ethyl- oder -propylether, Dipropylenglykolmonomethyl- oder - ethylether, Diisopropylenglykolmonomethyl- oder -ethylether, Methoxy-, Ethoxy- oder Butoxytriglykol, i-Butoxyethoxy-2-propanol, 3-Methyl-3-methoxybutanol, Propylen-glykol-t-butylether sowie Mischungen dieser Lösemittel.

Als Schauminhibitoren oder Entschäumer der Komponente e) kommen beispielsweise Seifen, Paraffine oder Silikonöle in Betracht, die gegebenenfalls auf Trägermaterialien aufgebracht sein können.

Geeignete Basen der Komponente e) sind insbesondere die zuvor bei den Buildern genannten Carbonate.

In einer alternativ bevorzugten Ausführungsform der Erfindung sind die erfindungsgemäßen Wasch- und Reinigungsmittel gelförmig. Bevorzugt handelt es sich bei den gelförmigen Wasch- und Reinigungsmitteln um gelförmige Geschirrspülmittel, worunter gelförmige Maschinengeschirrspülmittel besonders bevorzugt sind. Insbesondere handelt es sich um gelförmige Geschirrspülmittel, bevorzugt Maschinengeschirrspülmittel, mit Klarspülfunktion.

Unter gelförmigen Wasch- und Reinigungsmitteln versteht man fluide Mittel, die bei Raumtemperatur (20 °C) eine Viskosität aufweisen, die höher ist als die von Wasser, die aber noch soweit fließfähig sind, dass sie mit üblichen Dosierhilfsmitteln problemlos dosiert werden können. Vorzugsweise weisen die erfindungsgemäßen gelförmigen Wasch- und Reinigungsmittel eine Viskosität von 0,5 bis 100, besonders bevorzugt von 0,5 bis 50 und insbesondere von 1 bis 30 Pa·s bei 20 °C auf.

Das erfindungsgemäße gelförmige Geschirrspülmittel umfasst vorzugsweise folgende Bestandteile:
a) wenigstens eine Verbindung der Formel I;
b) wenigstens einen Builder (auch als Sequestrierungsmittel, Gerüststoff, Komplexbildner, Chelator, Chelatisierungsmittel oder Enthärter bezeichnet);
c) gegebenenfalls wenigstens ein Enzym;
d) gegebenenfalls wenigstens ein Bleichmittel;
e1) Wasser;
e2) wenigstens einen Verdicker; und
e3) gegebenenfalls wenigstens einen weiteren Zusatzstoff, der vorzugsweise ausgewählt ist unter Tensiden, die von a) verschieden sind, Basen, Korrosionsinhibitoren, Entschäumern, Farbstoffen, Duftstoffen, Füllstoffen, Löslichkeitsvermittlern und organischen Lösemitteln.

Vorzugsweise sind diese Bestandteile in folgenden Mengenverhältnissen im erfindungsgemäßen gelförmigen Geschirrspülmittel enthalten:
a) wenigstens eine Verbindung der Formel I: von 0,1 bis 20 Gew.-%;
b) wenigstens ein Builder: von 5 bis 80 Gew.-%;
c) wenigstens ein Enzym: von 0 bis 8 Gew.-%;
d) wenigstens ein Bleichmittel: von 0 bis 30 Gew.-%;
e1) Wasser: von 10 bis 90 Gew.-%;
e2) wenigstens ein Verdicker: von 0,1 bis 8 Gew.-%; und
e3) wenigstens ein weiterer Zusatzstoff: von 0 bis 25 Gew.-%.

Die Gew.-%-Angaben beziehen sich dabei auf das Gesamtgewicht des Geschirrspülmittels. Die Gewichtsmengen von a) bis e3) ergänzen sich zu 100 Gew.-%.

Besonders bevorzugt umfasst das erfindungsgemäße Geschirrspülmittel wenigstens ein Enzym.

Besonders bevorzugt sind die oben genannten Bestandteile in folgenden Mengenverhältnissen im erfindungsgemäßen gelförmigen Geschirrspülmittel enthalten:
a) wenigstens eine Verbindung der Formel I: von 0,1 bis 10 Gew.-%;
b) wenigstens ein Builder: von 5 bis 60 Gew.-%;
c) wenigstens ein Enzym: von 0,1 bis 6 Gew.-%;
d) wenigstens ein Bleichmittel: von 0 bis 30 Gew.-%; und
e1) Wasser: von 10 bis 90 Gew.-%;
e2) wenigstens ein Verdicker: von 0,1 bis 6 Gew.-%; und
e3) wenigstens ein weiterer Zusatzstoff: von 0 bis 25 Gew.-%.

Die Gew.-%-Angaben beziehen sich dabei auf das Gesamtgewicht des Geschirrspülmittels. Die Gewichtsmengen von a) bis e3) ergänzen sich zu 100 Gew.-%.

Stärker bevorzugt sind die oben genannten Bestandteile in folgenden Mengenverhältnissen im erfindungsgemäßen gelförmigen Geschirrspülmittel enthalten:
a) wenigstens eine Verbindung der Formel I: von 0,1 bis 10 Gew.-%;
b) wenigstens ein Builder: von 5 bis 40 Gew.-%;
c) wenigstens ein Enzym: von 0,1 bis 6 Gew.-%;
d) wenigstens ein Bleichmittel: von 0 bis 25 Gew.-%; und
e1) Wasser: von 20 bis 80 Gew.-%;
e2) wenigstens ein Verdicker: von 0,3 bis 5 Gew.-%; und
e3) wenigstens ein weiterer Zusatzstoff: von 0 bis 25 Gew.-%.

Die Gew.-%-Angaben beziehen sich dabei auf das Gesamtgewicht des Geschirrspülmittels. Die Gewichtsmengen von a) bis e3) ergänzen sich zu 100 Gew.-%.

Die Verdicker dienen dazu, dem erfindungsgemäßen Geschirrspülmittel die erwünschte Viskosität zu verleihen.

Geeignet sind grundsätzlich jedwede bekannte Verdicker (Rheologiemodifizierungsmittel), sofern sie keinen negativen Einfluss auf die Wirkung des Geschirrspülmittels ausüben. Geeignete Verdicker können sowohl natürlichen Ursprungs als auch synthetischer Natur sein.

Beispiele für Verdicker natürlichen Ursprungs sind Xanthan, Johannisbrotkernmehl, Guarmehl, Carrageen, Agar, Tragant, Gummi arabicum, Alginate, modifizierte Stärken, wie Hydroxyethylstärke, Stärkephosphatester oder Stärkeacetate, Dextrine, Pektine und Cellulosederivate, wie Carboxymethylcellulose, Hydroxyethylcellulose, Hydroxypropylcellulose, Hydroxypropylmethylcellulose, Methylcellulose und dergleichen.

Verdicker natürlichen Ursprungs sind auch anorganische Verdicker, wie Polykieselsäuren und Tonmineralien, z.B. Schichtsilikate, wie auch die bei den Buildern genannten Silikate.

Beispiele für synthetische Verdicker sind Polyacryl- und Polymethacrylverbindungen, wie (teil)vernetzte Homopolymere der Acrylsäure, beispielsweise mit einem Allylether von Saccharose oder Pentaerythrit oder mit Propylen vernetzte Homopolymere der Acrylsäure (Carbomer), z.B. die Carbopol®-Marken von BF Goodridge (z.B. Carbopol® 676, 940, 941, 934 und dgl.) oder die Polygel®-Marken von 3V Sigma (z.B. Polygel® DA), Copolymere ethylenisch ungesättigter Mono- oder Dicarbonsäuren, beispielsweise Terpolymere von Acrylsäure, Methacrylsäure oder Maleinsäure mit Methyl- oder Ethylacrylat und einem (Meth)Acrylat, das sich von langkettigen ethoxylierten Alkoholen ableitet, beispielsweise die Acusol®-Marken von Rohm & Haas (z.B. Acusol® 820 oder 1206A), Copolymere von zwei oder mehr Monomeren, die ausgewählt sind unter Acrylsäure, Methacrylsäure und ihren C₁-C₄-Alkylestern, z.B. Copolymere von Methacrylsäure, Butylacrylat und Methylmethacrylat oder von Butylacrylat und Methylmethacrylat, z.B. die Aculyn®- und Acusol®-Marken von Rohm & Haas (z.B. Aculyn® 22, 28 oder 33 und Acusol® 810, 823 und 830), oder vernetzte hochmolekulare Acrylsäurecopolymere, beispielsweise mit einem Allylether von Saccharose oder Pentaerythrit vernetzte Copolymere von C₁₀-C₃₀-Alkylacrylaten mit einem oder mehreren Comonomeren, die ausgewählt sind unter Acrylsäure, Methacrylsäure und ihren C₁-C₄-Alkylestern (z.B. Carbopol® ETD 2623, Carbopol® 1382 oder Carbopol® AQUA 30 von Rohm & Haas).

Beispiele für synthetische Verdicker sind weiterhin Umsetzungsprodukte von Maleinsäurepolymeren mit ethoxylierten langkettigen Alkoholen, z.B. die Surfonic L-Serie von Texaco Chemical Co. oder Gantrez AN-119 von ISP; Polyethylenglykole, Polyamide, Polyimine und Polycarbonsäuren.

Geeignet sind auch Gemische der o.g. Verdicker.

Bevorzugte Verdicker sind Xanthane und die oben genannten Polyacryl- und Polymethacrylverbindungen.

Bezüglich geeigneter und bevorzugter Komponenten a) bis d) und e3) wird auf die obigen Ausführungen Bezug genommen.

Die erfindungsgemäß eingesetzten Verbindungen I besitzen keine bzw. keine starke Neigung zur Schaumbildung, sie haben eine gute belagsinhibierende Wirkung auf damit gewaschenem Geschirr und sie sind nicht hydrolyselabil. Sie lassen sich außerdem gut in festen Wasch- und Reinigungsmitteln formulieren.

Die Erfindung betrifft außerdem Verbindungen der Formel I.1

R-S(O)ₓ-[-CH₂CH₂-O-]ₙ-CH₂CH₂-S(O)_{y}-R' (I.1)

worin
R und R' unabhängig voneinander für C₆-C₁₈-Alkyl stehen;
n für eine Zahl von 11 bis 150 steht; und
x und y für 0, 1 oder 2 stehen;
wobei x und y nicht gleichzeitig für 0 stehen.

In den erfindungsgemäßen Verbindungen l.1 stehen R und R' unabhängig voneinander vorzugsweise für C₈-C₁₆-Alkyl, besonders bevorzugt für C₈-C₁₄-Alkyl und insbesondere für C₁₀-C₁₄-Alkyl.

Bevorzugt stehen R und R' für einen linearen Alkylrest.

Bevorzugt haben R und R' die gleiche Bedeutung.

Speziell stehen R und R' für C₁₂-Alkyl, insbesondere für n-Dodecyl.

n steht vorzugsweise für eine Zahl von 15 bis 100, besonders bevorzugt von 20 bis 80, stärker bevorzugt von 25 bis 45 und insbesondere von 30 bis 40.

x und y stehen in Verbindungen I.1 unabhängig voneinander vorzugsweise für 1 oder 2. Besonders bevorzugt stehen beide für 1 oder beide für 2.

Die Erfindung betrifft außerdem Verbindungen der Formel I.2

R-S(O)ₓ-[-CH₂CH₂-O-]-ₙ-CH₂CH₂-S(O)_{y}-R' (I.2)

worin
R und R' unabhängig voneinander für C₈-C₁₈-Alkyl, vorzugsweise für C₁₀-C₁₈-Alkyl und insbesondere für C₁₂-C₁₈-Alkyl stehen;
n für eine Zahl von 11 bis 150 steht; und
x und y für 0, 1 oder 2 stehen

Bevorzugt stehen in Verbindungen I.2 R und R' für einen linearen Alkylrest.

Bevorzugt haben R und R' die gleiche Bedeutung.

n steht vorzugsweise für eine Zahl von 15 bis 100, besonders bevorzugt von 20 bis 80, stärker bevorzugt von 25 bis 45 und insbesondere von 30 bis 40.

In Verbindungen I.2 stehen x und y unabhängig voneinander vorzugsweise für 0 oder 1. Besonders bevorzugt stehen beide für 0 oder beide für 1.

Bezüglich geeigneter Herstellungsverfahren für Verbindungen I.1 und I.2 wird auf die obigen Ausführungen verwiesen.

Die Anmeldung wird nun durch die folgenden, nicht limitierenden Beispiele weiter veranschaulicht.

### Beispiele

### 1. Synthesebeispiele

### 1.1 Herstellung von 2-Hydroxydodecylether - PEG 1000 - 2-hydroxydodecylether (Vergleichsverbindung A)

In einem 2l Autoklaven der Firma Mettler wurde das zu alkoxylierende Polyethylenglykol PEG 1000 (250 g, 1,0 mol eq) mit einer wässrigen KOH-Lösung, die 50 Gew.-% KOH enthielt (0.2 Gew.-% KOH bezogen auf den Gesamtansatz, 1,4g KOH 50%ig), versetzt. Dabei betrug die Menge an KOH 0,2 Gew.-% des herzustellenden Produktes. Unter Rühren wurde die Mischung bei 100 °C und 20 mbar für 2 h entwässert. Anschließend wurde dreimal mit N₂ gespült, ein Vordruck von ca. 1,5 bar N₂ eingestellt und die Temperatur auf 140 °C erhöht. Dodecenoxid (92,15g, 2,0 mol eq.) wurde so zudosiert, dass die Temperatur zwischen 135 bis 145 °C blieb. Anschließend wurde 24 h bei 140 °C nachgerührt, mit N₂ gespült, auf 80 °C abgekühlt und der Reaktor entleert. Das Rohprodukt wurde am Rotationsverdampfer 2 h bei 100 °C (<20 mbar Vakuum) entgast. Dieses basische Rohprodukt wurde mit handelsüblichen Mg-Silikaten entionisiert, welche anschließend abfiltriert werden (Ausbeute: 99 %). Das helle Produkt wurde mit Hilfe eines ¹H-NMR-Spektrums in CDCl₃ und einer Gel-Permeations-Chromatographie identifiziert.

### 1.2 Herstellung von 2,2'-Di-(n-dodecylthio)-PEG 1500 (erfindungsgemäße Verbindung B)

In einer 500 mL Apparatur mit Wasserauskreiser wurden ein Polyethylenglykol mit Molekulargewicht 1500 (150 g, 0,1 mol), para-Toluolsulfonsäure (0,005 mol, 0,95 g) und Toluol (ca. 150 mL) vorgelegt. Die Reaktionsmischung wurde zum Rückfluss erhitzt. Dodecylmercaptoethanol (0,25 mol, 61,6 g) wurde zugetropft. Das entstehende Wasser (Dest.: 3,8 ml/ Th. Dest.: 4,5 ml) wurde über einen Wasserauskreiser über Nacht entfernt. Die Reaktionsmischung wurde abgekühlt, mit Natriumcarbonat neutralisiert (0,005 mol, 0,53 g), filtriert und das Lösemittel wurde bei 100 °C und 6 mbar vollständig entfernt. Man erhielt 165,4 g eines weißen festen Produktes. Die Struktur wurde mit TAl-NMR (93 % umgesetzte OH-Gruppen) bestimmt. Restmengen an Toluol (<0,5%) und Dodecylmercaptoethanol (0,3%) wurden über GC ermittelt und der PEG Gehalt (2,5 %) wurde mit HPTLC gemessen.

### 1.3 Herstellung von 2,2'-Di-(n-dodecylsulfoxy)-PEG 1500 (erfindungsgemäßes Produkt C)

In einem 250 mL Kolben wurde das Produkt aus Beispiel 1.2 (0,03 mol, 58,7 g) in Wasser vorgelegt und auf 70 °C erwärmt. Wasserstoffperoxid (50%ig) (0,06 mol, 4,1 g) wurde zugetropft. Die Reaktionsmischung wurde bei 70 °C 2 Stunden lang nachgerührt, dann abgekühlt und bei 100 °C und 6 mbar eingeengt. Man erhielt 54 g der Titelverbindung. Die Abwesenheit von Wasserstoffperoxid wurde per IR geprüft und der Umsatz über ¹H-NMR (86 %) bestimmt.

### 1.4 Herstellung von 2,2'-Di-(n-octylthio)-PEG 1500 (erfindungsgemäßes Produkt D)

In einer 500 mL Apparatur mit Wasserauskreiser wurden ein Polyethylenglykol mit Molekulargewicht 1500 (150 g, 0,1 mol), para-Toluolsulfonsäure (0,005 mol, 0,95 g) und Toluol (ca. 150 mL) vorgelegt. Die Reaktionsmischung wurde zum Rückfluss erhitzt. Octylmercaptoethanol (0,2 mol, 38,7 g) wurde zugetropft. Das entstehende Wasser (Dest.: 3,6ml/ Dest. Th.: 3,6ml) wurde über einen Wasserauskreiser über Nacht ausgekreist. Die Reaktionsmischung wurde abgekühlt, mit Natriumcarbonat neutralisiert (0,005 mol, 0,53 g), filtriert und das Lösemittel wurde bei 100 °C und 6 mbar vollständig entfernt. Man erhielt 172 g eines weißen festen Produktes. Die Struktur wurde mit TAI-NMR (83 % umgesetzte OH-Gruppen) bestimmt. Restmengen an Toluol (<0,5%) und Octylmercaptoethanol (0,7%) wurden über GC ermittelt und der PEG Gehalt (7 %) wurde mit HPTLC gemessen.

### Methodenbeschreibung für TAI-NMR:

Um im ¹H NMR-Spektrum überlagerte Signale von primären und/oder sekundären Alkoholen (quantitativ) bestimmen zu können, wird eine Probe in CDCl₃ mit einem Überschuss an TAI (Trichloracetylisocyanat) versetzt. Das Isocyanat reagiert dabei sofort mit den Alkoholgruppen zum Carbamat ab. Die darin enthaltenen Verbindungen Cl₃CC(O)NHC(O)-*OCH₂R* bzw. Cl₃CC(O)NHC(O)-*OCH*-RR' weisen im ¹H NMR-Spektrum unterschiedliche, typische Verschiebungen für OCH₂ und OCHRR' auf. Der typische Verschiebungsbereich für "primäre Carbamate" liegt bei 4.0 bis 4.5 ppm, während derjenige für "sekundäre Carbamate" bei 5.0 bis 5.3 ppm liegt.

### 2 Anwendungsbeispiele

### 2.1 Schaumvolumen in der Geschirrspülmaschine

Das Schaumvolumen wurde indirekt durch die Messung der Schaumbildung über die Drehzahl des Sprüharmes der Geschirrspülmaschine bestimmt. Hierfür wurden 10 ml verrührtes Hühnerei, 19 g eines Basisgeschirrspülmittels (48 Teile Natriummetasilikat x 5H₂O, 45 Teile Natriumtriphosphat, 5 Teile Natriumcarbonat) und 1 g des Tensids (A-D) in die Spülmaschine (Miele Desinfektor G 7735 CD MCU; Programm-Steuergerät MCU-Version S04.01) gegeben. Bei unterschiedlichen Temperaturen wurde dann die Zahl der Umdrehungen des Sprüharms gemessen. Bei hohem Schaumniveau wird der Sprüharm abgebremst, bei niedrigem kann er mit höchst möglicher Geschwindigkeit (ca. 125 U/min) arbeiten. Die Spitzendrehzahl der Spülmaschine liegt gewöhnlich bei ca. 125 Upm, wenn kein Schaum vorliegt. Die Höchstdrehzahl in der Geschirrspülmaschine wurde im vorliegenden Versuch künstlich (durch Bohrungen am Sprüharm, Stellung der Düsen) eingestellt, um einen breiteren Range zu erhalten, der Produkte besser unterscheiden lässt.

Die Umdrehungsgeschwindigkeit wurde bei 40, 50 und 60°C gemessen. In folgender Tabelle sind die Rotorgeschwindigkeiten in U/min bei verschiedenen Temperaturen aufgelistet.

| **Temperatur** | **A** | **B** | **C** | **D** |
|---|---|---|---|---|
| 40 [°C] | 80 | 43 | 101 | 86 |
| 50 [°C] | 88 | 50 | 102 | 97 |
| 60 [°C] | 96 | 62 | 109 | 105 |

### 2.2 Klarspültest

Alle Beispiele wurden mit einer Grundformulierung folgender Zusammensetzung durchgeführt:
1 Gewichtsteil Protease (Ovozyme® 64 T)
0,2 Gewichtsteile Amylase (Stainzyme® 12 T)
3 Gewichtsteile Tensid
10 Gewichtsteile Polyacrylsäure mit Molgewicht 4000 g/mol (Sokalan® PA 25 CI)
10,5 Gewichtsteile Natriumpercarbonat
4 Gewichtsteile Tetraacetylethylendiamin
2 Gewichtsteile Natriumdisilikat (Britesil® H 265 LC)
18,8 Gewichtsteile Natriumcarbonat
33 Gewichtsteile Na-Citrat Dihydrat
15 Gewichtsteile Methylglycindiessigsäuretrinatriumsalz (Trilon® M)
0,5 Gewichtsteile 1-Hydroxyethan-(1,1-diphosphonsäure) (HEDP; Cublen® K 8514 GR)

Ovozyme® und Stainzyme® sind Markenzeichen der Novozymes, Sokalan® und Trilon® sind Markenzeichen der BASF SE, Britesil® ist ein Markenzeichen der PQ Corp., Cublen® ist ein Markenzeichen der Zschimmer&Schwarz Mohsdorf GmbH & Co KG. Alle Klarspülversuche wurden in einer Geschirrspülmaschine der Fa. Miele (Typ G1222 SCL) durchgeführt. Dabei wurde das Programm mit 50°C (R-time 2) für den Waschzyklus und 65°C für das Klarspülzyklus gewählt. Die Prüfungen wurden mit aufgehärtetem Wasser mit einer Wasserhärte von 21 °dH (Ca/Mg):HCO₃ (3:1):1.35 durchgeführt. Es wurde kein separater Klarspüler zugegeben und die eingebaute Wasserenthärtung (lonentauscher) nicht mit Regeneriersalz regeneriert. Die oben genannte Formulierung wurde jeweils mit 21 g dosiert. Zu jedem Spülgang wurden 100 g eines Ballastschmutzes bestehend aus Fett, Protein und Stärke zugegeben. Als Testgeschirr dienten in jedem Reinigungsgang Messer aus Cromargan, blaue Melaminteller und Trinkgläser. Zwischen den Spülzyklen wurde jeweils eine Stunde gewartet, davon 10 min bei geschlossener Tür, 50 min bei geöffneter Tür.

Nach Beendigung des sechsten Spülzyklus wurde das Geschirr visuell in einer abgedunkelten Kammer unter Licht hinter einer Lochblende abgemustert und nach einer Notenskala von 1 (= starke Rückstände) bis 10 (= keine Rückstände) hinsichtlich Flecken, Streifen und filmartiger Beläge beurteilt.

| **Benotung spotting** | **A** | **B** | **C** | **D** |
|---|---|---|---|---|
| Messer | 2 | 3 | 1 | 3 |
| Trinkglas | 1 | 1 | 2 | 1 |
| Melaminteller | 3 | 3 | 4 | 3 |
| Summe | 6 | 7 | 7 | 7 |

| **Benotung filming** | **A** | **B** | **C** | **D** |
|---|---|---|---|---|
| Messer | 6 | 6 | 5 | 8 |
| Trinkglas | 5 | 5 | 5 | 8 |
| Melaminteller | 6 | 9 | 9 | 10 |
| Summe | 17 | 20 | 19 | 26 |

## Patentansprüche

1. Verwendung von gegebenenfalls oxidierten Thioethern von Polyalkylenoxiden der Formel I
R-S(O)ₓ-[-CH₂CH₂-O-]ₙ-CH₂CH₂-S(O)_{y}-R' (I)
worin
R und R' unabhängig voneinander für C₆-C₁₈-Alkyl stehen;
n für eine Zahl von 11 bis 150 steht; und
x und y unabhängig voneinander für 0, 1 oder 2 stehen;
in Wasch- oder Reinigungsmitteln.

2. Verwendung nach Anspruch 1, wobei die Wasch- und Reinigungsmittel unter Geschirrspülmitteln ausgewählt sind.

3. Verwendung nach einem der vorhergehenden Ansprüche, wobei die gegebenenfalls oxidierten Thioether von Polyalkylenoxiden I als Klarspültenside eingesetzt werden.

4. Verwendung nach einem der vorhergehenden Ansprüche, wobei R und R' unabhängig voneinander für C₈-C₁₄-Alkyl und bevorzugt für C₁₀-C₁₄-Alkyl stehen.

5. Verwendung nach einem der vorhergehenden Ansprüche, wobei R und R' unabhängig voneinander für einen linearen Alkylrest stehen.

6. Verwendung nach einem der vorhergehenden Ansprüche, wobei R und R' die gleiche Bedeutung haben.

7. Verwendung nach einem der vorhergehenden Ansprüche, wobei n für eine Zahl von 25 bis 45 steht.

8. Verwendung nach einem der vorhergehenden Ansprüche, wobei x und y für 0 oder 1, vorzugsweise für 0 stehen.

9. Wasch- oder Reinigungsmittel, enthaltend wenigstens einen oxidierten Thioether von Polyalkylenoxiden der Formel I gemäß der Definition in einem der Ansprüche 1 und 4 bis 8.

10. Wasch- oder Reinigungsmittel gemäß Anspruch 9, bei dem es sich um ein Geschirrspülmittel handelt.

11. Wasch- oder Reinigungsmittel gemäß Anspruch 10, bei dem es sich um ein Maschinengeschirrspülmittel handelt.

12. Wasch- oder Reinigungsmittel gemäß Anspruch 10 oder 11, bei dem es sich um ein Geschirrspülmittel mit Klarspülfunktion handelt.

13. Wasch- oder Reinigungsmittel gemäß einem der Ansprüche 9 bis 12, das bei Raumtemperatur fest ist.

14. Wasch- oder Reinigungsmittel gemäß einem der Ansprüche 11 bis 13, umfassend folgende Bestandteile:
a) wenigstens ein gegebenenfalls oxidierter Thioether von Polyalkylenoxiden der Formel I: von 0,1 bis 20 Gew.-%;
b) wenigstens einen Builder: von 5 bis 80 Gew.-%;
c) gegebenenfalls wenigstens ein Enzym: von 0 bis 8 Gew.-%;
d) gegebenenfalls wenigstens ein Bleichmittel: von 0 bis 30 Gew.-%; und
e) gegebenenfalls wenigstens einen weiteren Zusatzstoff, der ausgewählt ist unter Tensiden, die von a) verschieden sind, Basen, Korrosionsinhibitoren, Entschäumern, Farbstoffen, Duftstoffen, Füllstoffen, Tablettierhilfsmitteln, Desintegrationsmitteln, Verdickern, Löslichkeitsvermittlern, organischen Lösemitteln und Wasser: von 0 bis 50 Gew.-%;
bezogen auf das Gesamtgewicht des Wasch- oder Reinigungsmittels.

15. Wasch- oder Reinigungsmittel gemäß einem der Ansprüche 9 bis 12 und 14, das bei Raumtemperatur gelförmig ist.

16. Wasch- oder Reinigungsmittel gemäß Anspruch 15, umfassend folgende Bestandteile:
a) wenigstens ein gegebenenfalls oxidierter Thioether von Polyalkylenoxiden der Formel I: von 0,1 bis 20 Gew.-%;
b) wenigstens einen Builder: von 5 bis 80 Gew.-%;
c) gegebenenfalls wenigstens ein Enzym: von 0 bis 8 Gew.-%;
d) gegebenenfalls wenigstens ein Bleichmittel: von 0 bis 30 Gew.-%;
e1) Wasser: von 10 bis 90 Gew.-%;
e2) wenigstens einen Verdicker: von 0,1 bis 8 Gew.-%; und
e3) gegebenenfalls wenigstens einen weiteren Zusatzstoff, der ausgewählt ist unter Tensiden, die von a) verschieden sind, Basen, Korrosionsinhibitoren, Entschäumern, Farbstoffen, Duftstoffen, Füllstoffen, Löslichkeitsvermittlern und organischen Lösemitteln: von 0 bis 25 Gew.-%;
bezogen auf das Gesamtgewicht des Wasch- oder Reinigungsmittels.

17. Oxidierter Thioether von Polyalkylenoxiden der Formel I.1
R-S(O)ₓ-[-CH₂CH₂-O-]ₙ-CH₂CH₂-S(O)_{y}-R' (I.1)
worin
R und R' unabhängig voneinander für C₆-C₁₈-Alkyl stehen;
n für eine Zahl von 11 bis 150 steht; und
x und y für 0, 1 oder 2 stehen;
wobei x und y nicht gleichzeitig für 0 stehen.

18. Gegebenenfalls oxidierter Thioether von Polyalkylenoxiden der Formel I.2
R-S(O)ₓ-[-CH₂CH₂-O-]ₙ-CH₂CH₂-S(O)_{y}-R' (I.2)
worin
R und R' unabhängig voneinander für C₈-C₁₈-Alkyl, vorzugsweise C₁₀-C₁₈-Alkyl stehen;
n für eine Zahl von 11 bis 150 steht; und
x und y für 0, 1 oder 2 stehen.

19. Verfahren zur Herstellung von gegebenenfalls oxidierten Thioethern von Polyalkylenoxiden der Formeln I, I.1 oder I.2 gemäß der Definition in einem der Ansprüche 1, 4 bis 8, 17 oder 18, bei dem man ein Polyethylenglykol der Formel
HO-⁅CH₂CH₂-O⁆ₙ₋₂-CH₂CH₂-OH
mit einem Alkylmercaptoethanolderivat der Formel R-S-CH₂CH₂-OH oder R'-S-CH₂CH₂-OH oder einem Gemisch davon (wenn R und R' verschieden sind) umsetzt, wobei man für den Fall, dass R und R' verschieden sind, das Polyethylenglykol mit den verschiedenen Alkylmercaptoethanolderivaten der Formeln R-S-CH₂CH₂-OH und R'-S-CH₂CH₂-OH gleichzeitig oder nacheinander umsetzen kann.

## Claims

1. The use of optionally oxidized thioethers of polyalkylene oxides of the formula I
R-S(O)ₓ-⁅CH₂CH₂-O⁆ₙ-CH₂CH₂-S(O)_{y}-R' (I)
in which
R and R' are each independently C₆-C₁₈-alkyl;
n is from 11 to 150; and
x and y are each independently 0, 1 or 2;
in washing or cleaning compositions.

2. The use according to claim 1, wherein the washing and cleaning compositions are selected from dishwashing compositions.

3. The use according to either of the preceding claims, wherein the optionally oxidized thioethers of polyalkylene oxides I are used as rinse aid surfactants.

4. The use according to any of the preceding claims, wherein R and R' are each independently C₈-C₁₄-alkyl and preferably C₁₀-C₁₄-alkyl.

5. The use according to any of the preceding claims, wherein R and R' are each independently a linear alkyl radical.

6. The use according to any of the preceding claims, wherein R and R' are the same.

7. The use according to any of the preceding claims, wherein n is from 25 to 45.

8. The use according to any of the preceding claims, wherein x and y are each 0 or 1, preferably 0.

9. A washing or cleaning composition comprising at least one oxidized thioether of polyalkylene oxides of the formula I as defined in any of claims 1 and 4 to 8.

10. The washing or cleaning composition according to claim 9, which is a dishwashing composition.

11. The washing or cleaning composition according to claim 10, which is a machine dishwashing composition.

12. The washing or cleaning composition according to claim 10 or 11, which is a dishwashing composition with rinse aid function.

13. The washing or cleaning composition according to any of claims 9 to 12, which is solid at room temperature.

14. The washing or cleaning composition according to any of claims 11 to 13, comprising the following constituents:
a) at least one optionally oxidized thioether of polyalkylene oxides of the formula I: from 0.1 to 20% by weight;
b) at least one builder: from 5 to 80% by weight;
c) optionally at least one enzyme: from 0 to 8% by weight;
d) optionally at least one bleach: from 0 to 30% by weight; and
e) optionally at least one further additive selected from surfactants other than a), bases, corrosion inhibitors, defoamers, dyes, fragrances, fillers, tableting aids, disintegrants, thickeners, solubilizers, organic solvents and water: from 0 to 50% by weight;
based on the total weight of the washing or cleaning composition.

15. The washing or cleaning composition according to any of claims 9 to 12 and 14, which is in gel form at room temperature.

16. The washing or cleaning composition according to claim 15, comprising the following constituents:
a) at least one optionally oxidized thioether of polyalkylene oxides of the formula I: from 0.1 to 20% by weight;
b) at least one builder: from 5 to 80% by weight;
c) optionally at least one enzyme: from 0 to 8% by weight;
d) optionally at least one bleach: from 0 to 30% by weight;
e1) water: from 10 to 90% by weight;
e2) at least one thickener: from 0.1 to 8% by weight; and
e3) optionally at least one further additive selected from surfactants other than a), bases, corrosion inhibitors, defoamers, dyes, fragrances, fillers, solubilizers and organic solvents: from 0 to 25% by weight;
based on the total weight of the washing or cleaning composition.

17. An oxidized thioether of polyalkylene oxides of the formula I.1
R-S(O)ₓ-⁅CH₂CH₂-O⁆ₙ-CH₂CH₂-S(O)_{y}-R' (I.1)
in which
R and R' are each independently C₆-C₁₈-alkyl;
n is from 11 to 150; and
x and y are each 0, 1 or 2;
where x and y are not both 0.

18. An optionally oxidized thioether of polyalkylene oxides of the formula I.2
R-S(O)ₓ-⁅CH₂CH₂-O⁆ₙ-CH₂CH₂-S(O)_{y}-R' (I.2)
in which
R and R' are each independently C₈-C₁₈-alkyl, preferably C₁₀-C₈-alkyl;
n is from 11 to 150; and
x and y are each 0, 1 or 2.

19. A process for preparing optionally oxidized thioethers of polyalkylene oxides of the formulae I, I.1 or I.2 as defined in any of claims 1, 4 to 8, 17 or 18, in which a polyethylene glycol of the formula
HO⁅CH₂CH₂-O⁆ₙ₋₂-CH₂CH₂-OH
is reacted with an alkylmercaptoethanol derivative of the formula R-S-CH₂CH₂-OH or R'-S-CH₂CH₂-OH or a mixture thereof (when R and R' are different), where, in the case that R and R' are different, the polyethylene glycol can be reacted simultaneously or successively with the different alkylmercaptoethanol derivatives of the formulae R-S-CH₂CH₂-OH and R'-S-CH₂CH₂-OH.

## Revendications

1. Utilisation de thioéthers éventuellement oxydés de polyoxydes d'alkylène de formule I
R-S(O)ₓ-[CH₂CH₂-O-]ₙ-CH₂CH₂-S(O)_{y}-R' (I)
dans laquelle
R et R' représentent indépendamment l'un de l'autre un alkyle en C₆-C₁₈ ;
n représente un nombre de 11 à 150 ; et
x et y représentent indépendamment l'un de l'autre 0, 1 ou 2 ;
dans des détergents.

2. Utilisation selon la revendication 1, dans laquelle les détergents sont choisis parmi les agents de lavage de la vaisselle.

3. Utilisation selon l'une quelconque des revendications précédentes, dans laquelle les thioéthers éventuellement oxydés de polyoxydes d'alkylène I sont utilisés en tant que tensioactifs de rinçage.

4. Utilisation selon l'une quelconque des revendications précédentes, dans laquelle R et R' représentent indépendamment l'un de l'autre un alkyle en C₈-C₁₄ et de préférence un alkyle en C₁₀-C₁₄.

5. Utilisation selon l'une quelconque des revendications précédentes, dans laquelle R et R' représentent indépendamment l'un de l'autre un radical alkyle linéaire.

6. Utilisation selon l'une quelconque des revendications précédentes, dans laquelle R et R' ont la même signification.

7. Utilisation selon l'une quelconque des revendications précédentes, dans laquelle n représente un nombre de 25 à 45.

8. Utilisation selon l'une quelconque des revendications précédentes, dans laquelle x et y représentent 0 ou 1, de préférence 0.

9. Détergent, contenant au moins un thioéther oxydé de polyoxydes d'alkylène de formule I selon la définition dans l'une quelconque des revendications 1 et 4 à 8.

10. Détergent selon la revendication 9, qui consiste en un agent de lavage de la vaisselle.

11. Détergent selon la revendication 10, qui consiste en un agent de lavage de la vaisselle en machine.

12. Détergent selon la revendication 10 ou 11, qui consiste en un agent de lavage de la vaisselle à fonction de rinçage.

13. Détergent selon l'une quelconque des revendications 9 à 12, qui est solide à température ambiante.

14. Détergent selon l'une quelconque des revendications 11 à 13, comprenant les constituants suivants :
a) au moins un thioéther éventuellement oxydé de polyoxydes d'alkylène de formule I : de 0,1 à 20 % en poids ;
b) au moins un adjuvant : de 5 à 80 % en poids ;
c) éventuellement au moins une enzyme : de 0 à 8 % en poids ;
d) éventuellement au moins un agent blanchissant : de 0 à 30 % en poids ; et
e) éventuellement au moins un additif supplémentaire, qui est choisi parmi les tensioactifs qui sont différents de a), les bases, les inhibiteurs de corrosion, les antimousses, les colorants, les parfums, les charges, les adjuvants de pastillage, les agents de désintégration, les épaississants, les solubilisants, les solvants organiques et l'eau : de 0 à 50 % en poids ;
par rapport au poids total du détergent.

15. Détergent selon l'une quelconque des revendications 9 à 12 et 14, qui est sous forme de gel à température ambiante.

16. Détergent selon la revendication 15, comprenant les constituants suivants :
a) au moins un thioéther éventuellement oxydé de polyoxydes d'alkylène de formule I : de 0,1 à 20 % en poids ;
b) au moins un adjuvant : de 5 à 80 % en poids ;
c) éventuellement au moins une enzyme : de 0 à 8 % en poids ;
d) éventuellement au moins un agent blanchissant : de 0 à 30 % en poids ;
e1) de l'eau : de 10 à 90 % en poids ;
e2) au moins un épaississant : de 0,1 à 8 % en poids ; et
e3) éventuellement au moins un additif supplémentaire, qui est choisi parmi les tensioactifs qui sont différents de a), les bases, les inhibiteurs de corrosion, les antimousses, les colorants, les parfums, les charges, les solubilisants et les solvants organiques : de 0 à 25 % en poids ;
par rapport au poids total du détergent.

17. Thioéther oxydé de polyoxydes d'alkylène de formule I.1
R-S(O)ₓ-[CH₂CH₂-O-]ₙ-CH₂CH₂-S(O)_{y}-R' (I.1)
dans laquelle
R et R' représentent indépendamment l'un de l'autre un alkyle en C₆-C₁₈ ;
n représente un nombre de 11 à 150 ; et
x et y représentent 0, 1 ou 2 ;
x et y ne représentant pas simultanément 0.

18. Thioéther éventuellement oxydé de polyoxydes d'alkylène de formule I.2
R-S(O)ₓ-[CH₂CH₂-O-]ₙ-CH₂CH₂-S(O)_{y}-R' (I.2)
dans laquelle
R et R' représentent indépendamment l'un de l'autre un alkyle en C₈-C₁₈, de préférence un alkyle en C₁₀-C₁₈ ;
n représente un nombre de 11 à 150 ; et
x et y représentent 0, 1 ou 2.

19. Procédé de fabrication de thioéthers éventuellement oxydés de polyoxydes d'alkylène de formule I, I.1 ou I.2 selon la définition dans l'une quelconque des revendications 1, 4 à 8, 17 ou 18, selon lequel un polyéthylène glycol de formule
HO-[CH₂CH₂-O-]ₙ₋₂-CH₂CH₂-OH
est mis en réaction avec un dérivé d'alkylmercaptoéthanol de formule R-S-CH₂CH₂-OH ou R'-S-CH₂CH₂-OH ou un mélange de ceux-ci (lorsque R et R' sont différents) ; lorsque R et R' sont différents, le polyéthylène glycol pouvant être mis en réaction simultanément ou successivement avec les dérivés d'alkylmercaptoéthanol des formules R-S-CH₂CH₂-OH et R'-S-CH₂CH₂-OH différents.
